# EUROPEAN PATENT APPLICATION

(11) **EP 4 442 315 A2**
(43) Date of publication of application: **09.10.2024**
(21) Application number: 24189237.1
(22) Date of filing: 09.01.2020
(51) Int. Cl.: A61P 17/00

(54) **LTA4H INHIBITOR FOR THE TREATMENT OR PREVENTION OF HIDRADENITIS SUPPURATIVA**

(30) Priority: 11.01.2019 EP 19151459; 06.09.2019 US 201962896923 P
(62) Divisional of application: 20701236.0
(71) Applicant: Novartis AG, 4056 Basel (CH)
(72) Inventor: LOESCHE, Christian, 4002 Basel (CH); PENNO, Carlos, 4002 Basel (CH); ROEHN, Till, 4002 Basel (CH); WIECZOREK, Grazyna, 4002 Basel (CH)
(74) Representative: Binet Cross, Sophie

(57) **Abstract**

The present disclosure relates to methods for treating Hidradenitis Suppurativa using a LTA4H inhibitor. Also disclosed herein are LTA4H inhibitors, for treating Hidradenitis Suppurativa patients, as well as medicaments, dosing regimens, pharmaceutical formulations, combinations, dosage forms, and kits for use in the disclosed uses and methods.

## Description

### TECHNICAL FIELD

The present disclosure relates to methods for treating Hidradenitis suppurativa using leukotriene A4 hydrolase (LTA4H) inhibitors.

### BACKGROUND OF THE INVENTION

Hidradenitis suppurativa (HS) (also referred to as acne inversa or Verneuil's disease) is a chronic, recurring, inflammatory disease characterized by deep-seated nodules, sinus tracts, and abscesses that lead to fibrosis in the axillary, inguinal, breast-fold, and anogenital regions. (Revuz and Jemec (2016) Dermatol Clin 34:1-5; Jemec GB. (2012) N Engl J Med 366:158-64). It is associated with substantial pain and comorbidities, including metabolic, psychiatric, and autoimmune disorders, as well as an increased risk of skin cancer. (Revuz (2016); Shlyankevich et al. (2014) J Am Acad Dermatol 71:1144-50; Kohorst et al (2015) J Am Acad Dermatol 73:S27-35; Wolkenstein et al. (2007) J Am Acad Dermatol 56:621-3).

Reported prevalence rates of HS vary from <1% to 4% of the population. [Shlyankevich et al. (2014); Cosmatos et al. (2013) J Am Acad Dermatol 68:412-9; Davis et al. (2015) Skin Appendage Disord 1:65-73; Revuz et al. (2008) J Am Acad Dermatol 59:596-601; McMillan K. (2014) Am J Epidemiol 179:1477-83; Garg et al. (2017) J Am Acad Dermatol, 77(1):118-122; Jemec et al. (1996) J Am Acad Dermatol 35:191-4]. However, the true prevalence is difficult to ascertain because HS is underdiagnosed, and estimates fluctuate with study design, population, and geographic location. [Miller et al. (2016) Dermatol Clin 34:7-16]. Although the National Institutes of Health (NIH) does not classify HS as a rare disease, experts generally consider the prevalence of the disease to be <1% of the United States (US) population. [Cosmatos et al. (2013); Genetic and Rare Diseases Information Center. National Institutes of Health. Hidradenitis suppurativa. Available at: //rarediseases.info.nih.gov/diseases/6658/hidradenitis-suppurativa. Accessed March 20, 2017; Gulliver et al. (2016) Rev Endocr Metab Disord 17:343-51; Garg et al. 2007].

Current treatment for HS consists of topical and/or systemic antibiotics, hormonal interventions, retinoids, and, in selected cases, immunosuppressants, biologics such as the tumor necrosis factor [TNF] inhibitor monoclonal antibody adalimumab, the only approved drug in HS and often as last resort large surgical excision. [Gulliver et al. (2016); Zouboulis et al. (2015) J Eur Acad Dermatol Venereol 29:619-4414-16; Kimball et al. (2016) N Engl J Med 375:422-34]. As the lesions are painful, the patients need very often analgesics and pain relieve.

However, symptom control and lesion resolution are inconsistent among treatments and randomized controlled clinical trial results providing evidence-based data are lacking for most treatments and only adalimumab is approved. While antibiotic therapy is used for long-term treatment for months and even years, antibiotic therapy may thus results in resurgence of antibacterial resistance. Oral treatment with retinoids poses teratogenicity concerns in a population that is sexually active and mostly female. Moreover, the effectiveness of inflammatory drugs, such as dapsone, fumarates and cyclosporine, is based on small case studies with varying results and these molecules are not systematically used. Because of these inconsistent outcomes, and the severity of the HS disease, HS patients utilize healthcare in high-cost settings (e.g., emergency department and inpatient care) more frequently than patients with other chronic inflammatory skin conditions. (Khalsa et al. (2016) J Am Acad Dermatol 73:609-14; Kirby et al. (2014) JAMA Dermatol 150:937-44). Because there is no medical cure for HS, and the disease is physically and psychologically debilitating, there is a clear unmet need to provide safe and effective long-term treatments for HS patients, in particular oral treatments.

### SUMMARY OF THE INVENTION

While the pathogenesis of HS is still not fully understood, this chronic skin disease is characterized by high numbers of neutrophils and macrophages in inflamed HS lesions. Neutrophils and macrophages seem to be key drivers of the pathomechanism of hidradenitis suppurativa [Shah et al (2017), "The critical role of macrophages in the pathogenesis of hidradenitis suppurativa". Inflamm. Res. 66(11): 931-945; [Lima et al (2016), "Keratinocytes and neutrophils are important sources of proinflammatory molecules in hidradenitis suppurativa." Br. J. Dermatol. 174: 514-21; Neutrophils and macrophages are the main producers and target cells of the inflammatory lipid mediator leukotriene B4 (LTB4) and the anti-inflammatory, pro-resolution mediator lipoxin A4 (LXA4) (Serhan et al 2008, "Resolving inflammation: dual anti-inflammatory and pro-resolution lipid mediators." Nat. Rev. Immunol. p. 349-61). The pro-inflammatory lipid mediator LTB4 is one of the most potent chemotactic molecules for neutrophils and monocytes and can stimulate and activate both neutrophil and macrophages [Marc Peters-Golden and William R Henderson (2007) "Leukotrienes", N Engl J Med 357:1841-1854]. Application of LTB4 to healthy skin causes influx of IL-17+ neutrophils, implicated in the pathomechanism of HS [Lima et al (2016), "Keratinocytes and neutrophils are important sources of proinflammatory molecules in hidradenitis suppurativa." Br. J. Dermatol. 174: 514-21.], formation of neutrophilic abscesses and keratinocyte hyperproliferation. [Hendriks et al (2014) "Cutaneous application of leukotriene B4 as an in vivo model of psoriasis-like skin inflammation: an immunohistological study), Skin Pharmacol Physiol 27 (3):120-6. Relevance of LTB4 was clinically demonstrated in related skin pathology inflammatory acne (Zouboulis et al 2003, "A new concept for acne therapy: a pilot study with zileuton, an oral 5-lipoxygenase inhibitor. Arch Dermatol" p. 668-70).

In house analysis of HS biopsies by lipidomics has revealed strong elevation of the lipid mediator LTB4 in lesional skin of HS patients as compared to healthy skin. Furthermore leukocytes in lesions were predominant and revealed strong expression of 5-lipoxygenase and intracellular localization of this enzyme at the perinuclear membrane, a unequivocal sign of 5-lipoxygenase pathway activation, leading to production of LTB4. (Christmas et al 1999, "Differential localization of 5- and 15- lipoxygenases to the nuclear envelope in RAW macrophages." J. Biol. Chem. p. 25594-8). Transcriptomics anlysis of lesions revealed overexpression of the 5-lipoxygenase pathway genes ALOX5 and LTA4H, the two enzymes responsible for LTB4 biosynthesis from arachidonic acid [Marc Peters-Golden and William R Henderson (2007) "Leukotrienes", N Engl J Med 357:1841-1854]. Inhibition of the final and rate limiting enzyme LTA4H of the 5-lipoxygenase pathway can prevent pro-inflammatory LTB4 biosynthesis but maintain or even elevate anti-inflammatory LXA4 biosynthesis. In addition, LXA4 elevation may have impact on fibrosis in addition to the anti-inflammatory effect of the drug (Börgeson et al 2011, "Lipoxin A4 and benzo-lipoxin A4 attenuate experimental renal fibrosis." FASEB J. p. 2967-79).

Compounds described in the invention inhibit LTA4H and thereby prevent the biosynthesis of pro-inflammatory leukotriene B4 (LTB4) but increases the generation of anti-inflammatory, resolution-enhancing lipoxin A4 (LXA4). Both lipid mediators are known to play an important role in the orchestration of neutrophilic inflammation. LTA4H inhibition represents a relevant mechanism for treatment of neutrophil-driven inflammatory conditions.

Therefore, the aim of the present invention is to provide novel method of treating or preventing Hidradenitis suppurativa (HS) in a subject in need thereof, comprising administering to said subject, an effective amount of a leukotriene A4 hydrolase (LTA4H) inhibitor.

More particularly, the invention pertains to a method of treating or preventing Hidradenitis suppurativa in a subject in need thereof, comprising administering to said subject a therapeutically effective amount of a compound of Formula (I): 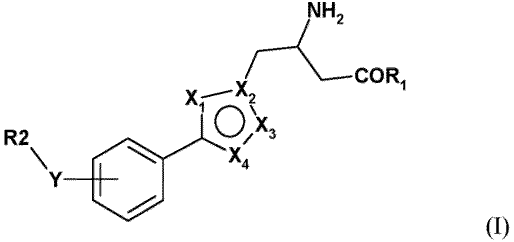 wherein,
R1 is OH or NH₂;
Y is O, S or CH₂;
X1, X2, X3 and X4 are N; or
X1, X2, X3 and X4 are selected from N, NH, C, CH and O with the proviso that at least two of
X1, X2, X3 or X4 are N or NH;
R2 is C₁-C₆ alkyl optionally substituted by phenyl; C₃-C₆ cycloalkyl; phenyl optionally being substituted by halogen, cyano, C₁-C₆ alkyl optionally substituted by halogen, C₁-C₆ alkoxy, or a 5-6 membered heteroaryl ring containing 1 to 3 heteroatoms selected from N, O and S; or a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen; or a pharmaceutically acceptable salt thereof.

Furthermore, the invention further provides a method of treating or preventing HS in a subject in need thereof, comprising administering to said subject a therapeutically effective amount of a LTA4H inhibitor with one or more therapeutic agents.

In another aspect of the invention, the present invention provides a LTA4H inhibitor for use in the treatment and/or prevention of HS, in a patient in need of such treatment and/or prevention. More particularly, the invention pertains to a compound of Formula (I) as described above for the use in the treatment and/or prevention of HS, in a patient in need of such treatment and/or prevention.

The invention further relates to combinations of a LTA4H inhibitor with one or more additional therapeutic agents, for use in the treatment or prevention of HS, in a patient in need of such treatment and/or prevention.

### BRIEF DESCRIPTION OF THE DRAWINGS

**Figure 1** provides an illustrative XRPD spectrum for the crystalline form of (*S*)-3-amino-4-(5-(4-((5-chloro-3-fluoropyridin-2-yl)oxy)phenyl)-2*H*-tetrazol-2-yl)butanoic acid in its free form, designated herein as Form B, showing degrees 20 (2-theta) on the X-axis and relative intensity on the Y-axis.
**Figure 2** provides an illustrative DSC for the free form of (*S*)-3-amino-4-(5-(4-((5-chloro-3-fluoropyridin-2-yl)oxy)phenyl)-2*H*-tetrazol-2-yl)butanoic acid, designated herein as Form B.
**Figure 3** provides an illustrative TGA for the free form of (*S*)-3-amino-4-(5-(4-((5-chloro-3-fluoropyridin-2-yl)oxy)phenyl)-2*H*-tetrazol-2-yl)butanoic acid, designated herein as Form B.
   More detailed listings of the XRPD peaks for each of form B is set forth in Table 1 below, in which the % relative intensity (I/I₀ x 100) is also provided. It should be understood that in the X-ray powder diffraction spectra or pattern that there is inherent variability in the values measured in degrees 2θ (°2θ) as a result of, for example, instrumental variation (including differences between instruments). As such, it should be understood that there is a variability of up to ± 0.2 °2Θ in XRPD peak measurements and yet such peak values would still be considered to be representative of a particular solid state form of the crystalline materials described herein. It should also be understood that other measured values from XRPD experiments and DSC/TGA experiments, such as relative intensity and water content, can vary as a result of, for example, sample preparation and/or storage and/or environmental conditions, and yet the measured values will still be considered to be representative of a particular solid state form of the crystalline materials described herein.

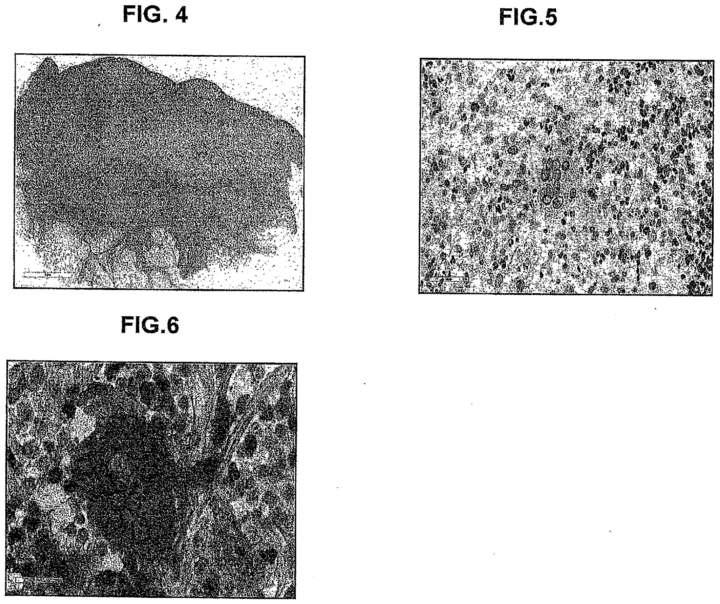

**Figure 4** provides analysis of lesions of HS patients by immunohistochemistry applying the H&E staining.
**Figure 5** provides an immunohistochemical analysis of HS lesional skin biospies
**Figure 6** A representative photomicrograph illustrating 5-Lipoxygenase localized at the nuclear membrane in multinucleated giant cell CD68+.

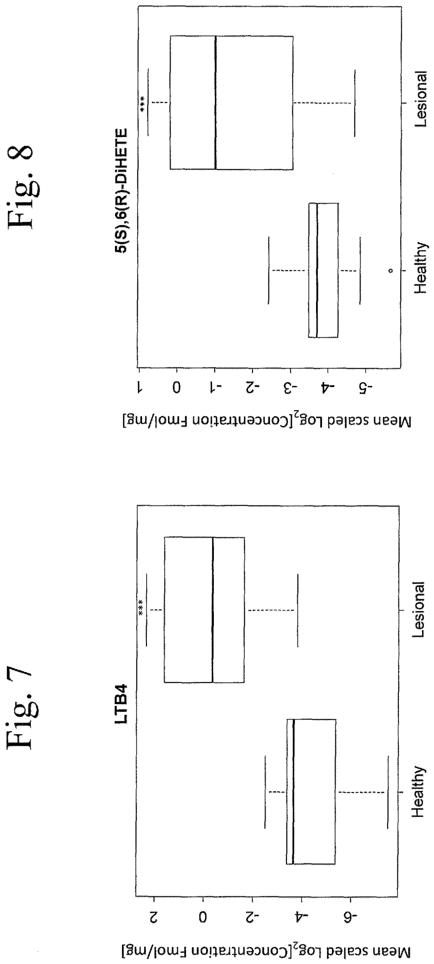

**Figure 7** provides LC-MS/MS analysis of lipids from biopsies of HS skin and skin of healthy volunteers for content of LTB4.
**Figure 8** provides LC-MS/MS analysis of lipids from biopsies of HS skin and skin of healthy volunteers for content of 5-HETE.

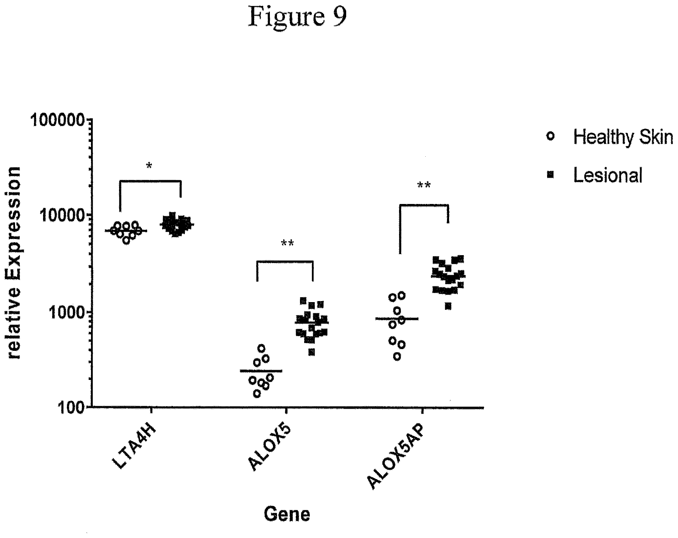

**Figure 9** provides transcriptomics analysis of HS skin biopsies versus healthy skin biopsies.

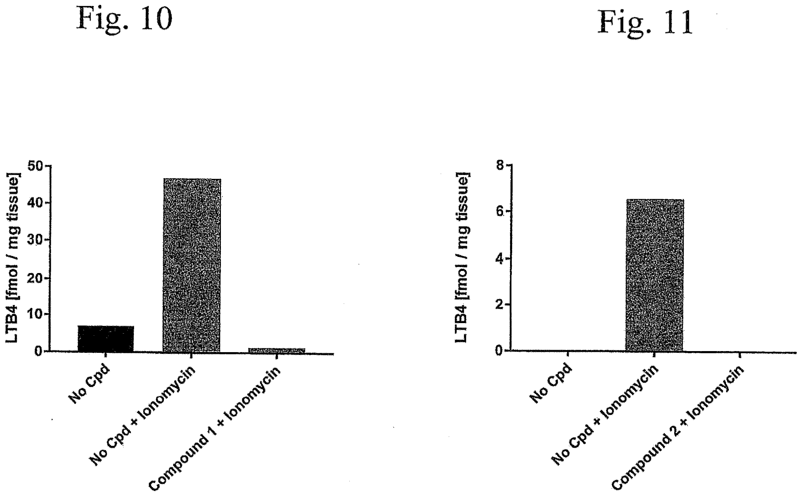

**Figure 10** Compound of Example 1 suppresses the biosynthesis of pro-inflammatory LTB4 in skin biopsies of HS patients as measured by LC-MS/MS.
**Figure 11** Compound of embodiment 3D suppresses the biosynthesis of pro-inflammatory LTB4 in skin biopsies of HS patients as measured by LC-MS/MS.

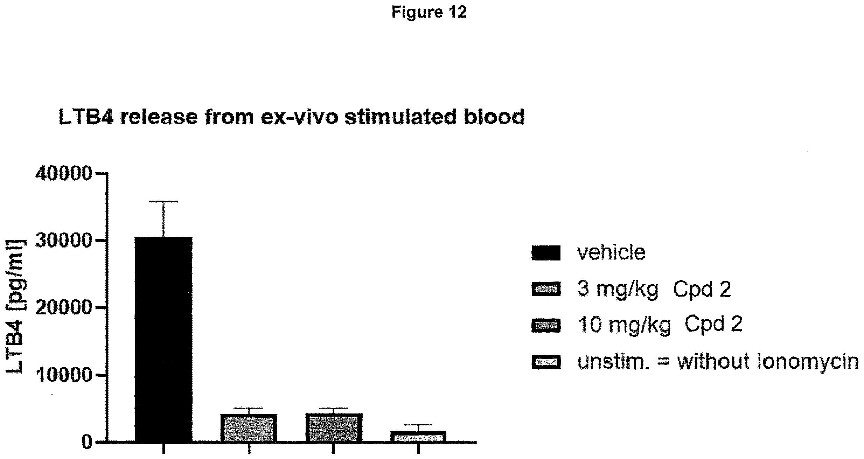

**Figure 12****:** Inhibition of LTB4 release from ex-vivo stimulated blood in mice treated with 3mg/kg or 10mg/kg of (*S*)-3-amino-4-(5-(4-(4-chlorophenoxy)-phenyl)-2*H*-tetrazol-2-yl)butanoic acid (Compound of embodiment 3D).

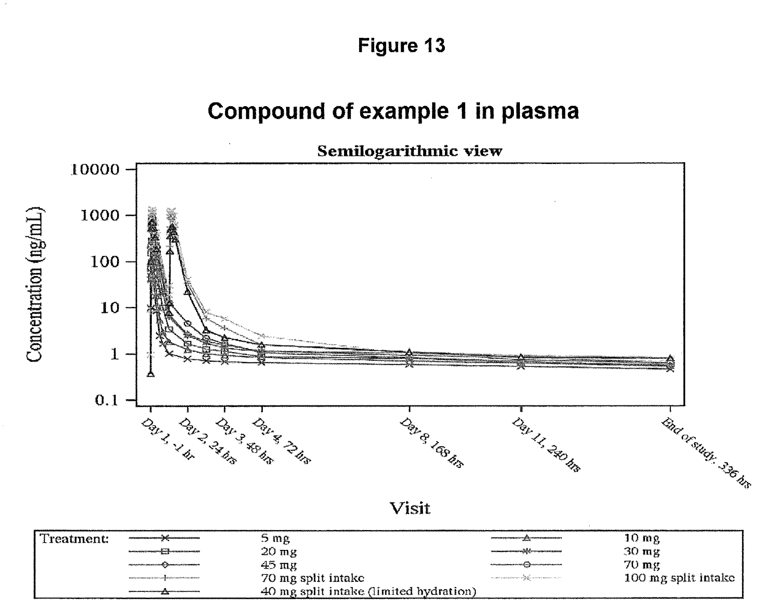

**Figure 13****:** Mean plasma concentration time profiles following single oral administration of (*S*)-3-amino-4-(5-(4-((5-chloro-3-fluoropyridin-2-yl)oxy)phenyl)-2*H*-tetrazol-2-yl)butanoic acid (Compound of example 1) at different doses.

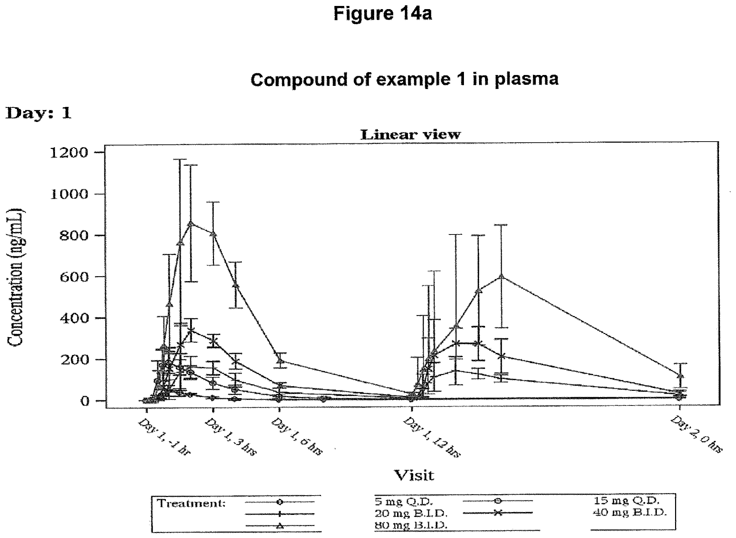

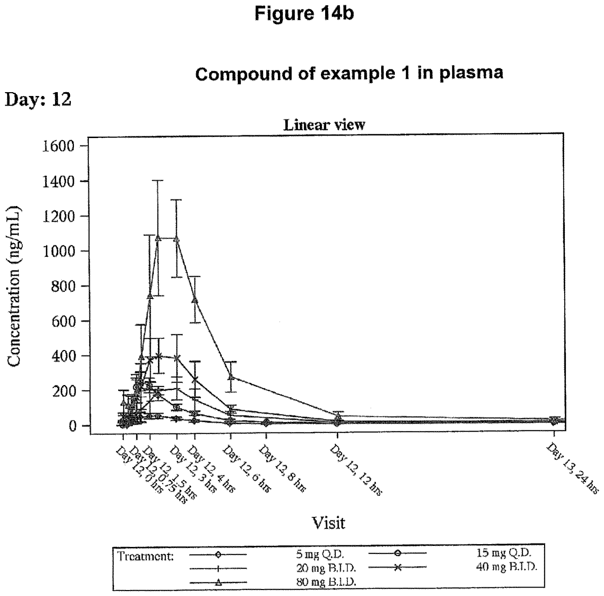

**Figure 14****:** Mean plasma concentration time profile following multiple oral administration of (*S*)-3-amino-4-(5-(4-((5-chloro-3-fluoropyridin-2-yl)oxy)phenyl)-2*H*-tetrazol-2-yl)butanoic acid (Compound of example 1) at different doses (Figure 14a: Day 1; Figure 14b: Day 12)

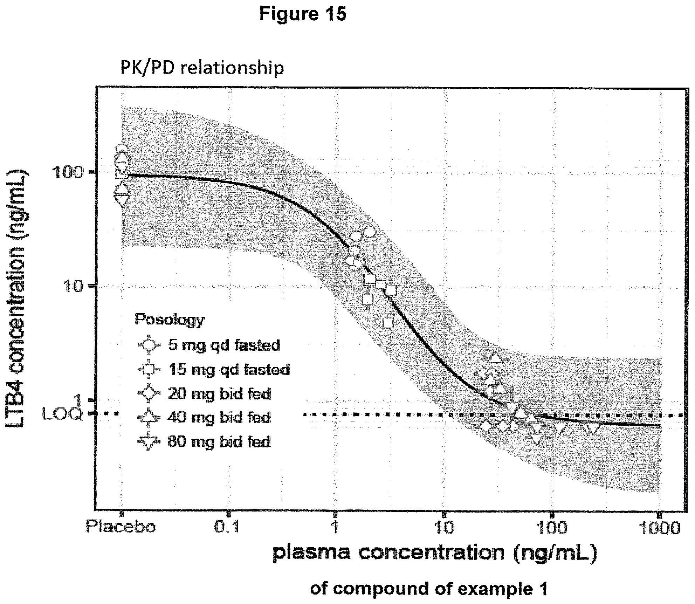

**Figure 15****:** LTB4 concentration in ex-vivo stimulated blood after oral administration of (*S*)-3-amino-4-(5-(4-((5-chloro-3-fluoropyridin-2-yl)oxy)phenyl)-2*H*-tetrazol-2-yl)butanoic acid (Compound of example 1) at measured plasma concentration of compound of example 1 in plasma (PK/PD relationship)

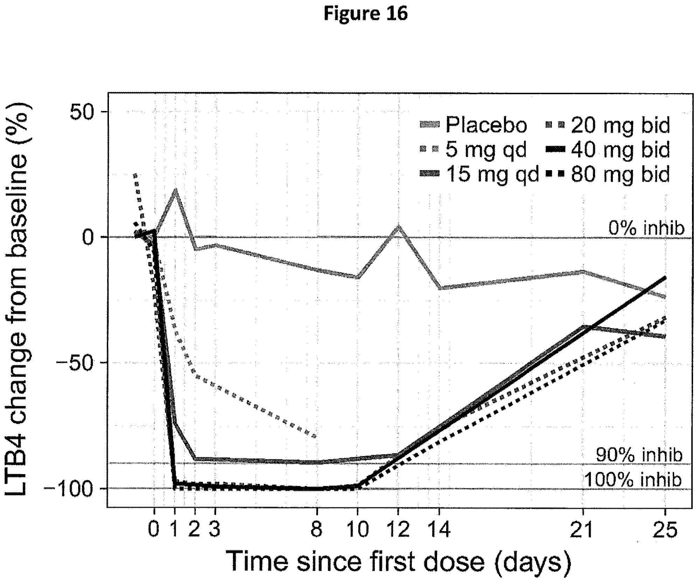

**Figure 16****:** LTB4 change from baseline (inhibition in blood) after multiple oral administration of (*S*)-3-amino-4-(5-(4-((5-chloro-3-fluoropyridin-2-yl)oxy)phenyl)-2*H*-tetrazol-2-yl)butanoic acid (Compound of example 1) at different dose measured at different time (days) since first dose.

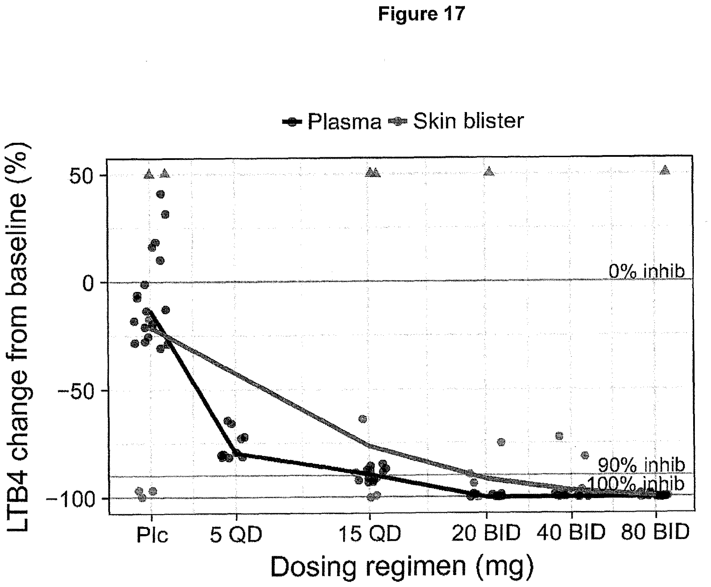

**Figure 17****:** LTB4 inhibition in skin and plasma after oral administration of (*S*)-3-amino-4-(5-(4-((5-chloro-3-fluoropyridin-2-yl)oxy)phenyl)-2*H*-tetrazol-2-yl)butanoic acid (Compound of example 1).

### DETAILED DESCRIPTION OF THE INVENTION

As used herein, the term "C₁-C₆ alkyl" refers to a fully saturated branched or unbranched hydrocarbon moiety having up to 6 carbon atoms. Unless otherwise provided, it refers to hydrocarbon moieties having 1 to 6 carbon atoms, 1 to 4 carbon atoms or 1 to 2 carbon atoms. Representative examples of alkyl include, but are not limited to, methyl, ethyl, *n*-propyl, *iso*propyl, *n*-butyl, *sec*-butyl, *iso*-butyl, *tert*-butyl, *n*-pentyl, isopentyl, neopentyl, *n*-hexyl and the like.

As used herein, the term "C₁-C₆ alkoxy" refers to alkyl-O-, wherein alkyl is defined herein above. Representative examples of alkoxy include, but are not limited to, methoxy, ethoxy, propoxy, 2-propoxy, butoxy, tert-butoxy, pentyloxy, hexyloxy, cyclopropyloxy-, cyclohexyloxy- and the like. Typically, alkoxy groups have about 1 to 6 carbon atoms, 1 to 4 carbon atoms or 1 to 2 carbon atoms.

As used herein, the term "C₁-C₆ alkyl optionally substituted by halogen" refers to C₁-C₆ alkyl as defined above which may be substituted by one or more halogens. Examples include, but are not limited to, trifluoromethyl, difluoromethyl, fluoromethyl, trichloromethyl, 2,2,2-trifluoroethyl, 1-fluoromethyl-2-fluoroethyl, 3-bromo-2-fluoropropyl and 1-bromomethyl-2-bromoethyl.

As used herein, the term "di-C₁₋₆alkylamino" refers to a moiety of the formula -N(Rₐ)-Rₐ where each Rₐ is a C₁₋₆alkyl, which may be the same or different, as defined above.

As used herein, the term "C₃-C₆ cycloalkyl" refers to saturated monocyclic hydrocarbon groups of 3-6 carbon atoms. Cycloalkyl may also be referred to as a carbocyclic ring and vice versa additionally referring to the number of carbon atoms present. Unless otherwise provided, cycloalkyl refers to cyclic hydrocarbon groups having between 3 and 6 ring carbon atoms or between 3 and 4 ring carbon atoms. Exemplary monocyclic hydrocarbon groups include, but are not limited to, cyclopropyl, cyclobutyl, cyclopentyl, and cyclohexyl.

As used herein, the term "halogen" or "halo" refers to fluoro, chloro, bromo, and iodo.

As used herein, the term "heteroaryl" refers to a 5-14 membered monocyclic- or bicyclic- or tricyclic-aromatic ring system, having 1 to 8 heteroatoms. Typically, the heteroaryl is a 5-10 membered ring system containing 1 to 4 hereroatoms selected from N, S or O (*e.g.,* 5-7 membered monocycle or an 8-10 membered bicycle) or a 5-7 membered ring system. Preferably, the term "heteroaryl" is a 5-7 membered monocycle. Typical heteroaryl groups include 2- or 3-thienyl, 2- or 3-furyl, 2- or 3-pyrrolyl, 2-, 4-, or 5-imidazolyl, 3-, 4-, or 5-pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2, 3-triazolyl, tetrazolyl, 2-, 3-, or 4-pyridyl, 3- or 4-pyridazinyl, 3-, 4-, or 5-pyrazinyl, 2-pyrazinyl, and 2-, 4-, or 5-pyrimidinyl.

The term "heteroaryl" also refers to a group in which a heteroaromatic ring is fused to one or more aryl, cycloaliphatic, or heterocyclyl rings, where the radical or point of attachment is on the heteroaromatic ring. Nonlimiting examples include 1-, 2-, 3-, 5-, 6-, 7-, or 8- indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8- purinyl, 1-, 2-, 3-, 4-, 6-, 7-, 8-, or 9-quinolizinyl, 2-, 3-, 4-, 5-, 6-, 7-, or 8-quinoliyl, 1-, 3-, 4-, 5-, 6-, 7-, or 8-isoquinoliyl, 1-, 4-, 5-, 6-, 7-, or 8-phthalazinyl, 2-, 3-, 4-, 5-, or 6-naphthyridinyl, 2-, 3-, 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4aH carbazolyl, 1-, 2-, 3-, 4-, 5-, 6-, 7-, or 8-carbzaolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1-, 2-, 3-, 4-, 5-, 6-, 7-, 8-, or 9-acridinyl, 1-, 2-, 4-, 5-, 6-, 7-, 8-, or 9-perimidinyl, 2-, 3-, 4-, 5-, 6-, 8-, 9-, or 10-phenathrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenoxazinyl, 2-, 3-, 4-, 5-, 6-, or l-, 3-, 4-, 5-, 6-, 7-, 8-, 9-, or 10- benzisoqinolinyl, 2-, 3-, 4-, or thieno[2,3-b]furanyl, 2-, 3-, 5-, 6-, 7-, 8-, 9-, 10 -, or 11-7H-pyrazino[2,3-c]carbazolyl,2-, 3-, 5-, 6-, or 7-2H- furo[3,2-b]-pyranyl, 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d] thiazolyl, 3-, 5-, or 8-pyrazino[2,3-d]pyridazinyl, 2-, 3-, 5-, or 6- imidazo[2,1-b] thiazolyl, 1-, 3-, 6-, 7-, 8-, or 9-furo[3,4-c]cinnolinyl, 1-, 2-, 3-, 4-, 5-, 6-, 8-, 9-, 10, or 11-4H-pyrido[2,3-c]carbazolyl, 2-, 3-, 6-, or 7-imidazo[1,2-b][1,2,4]triazinyl, 7-benzo[b]thienyl, 2-, 4-, 5-, 6-, or 7-benzoxazolyl, 2-, 4-, 5-, 6-, or 7-benzimidazolyl, 2-, 4-, 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzoxazinyl, 1-, 2-, 3-, 5-, 6-, 7-, 8-, 9-, 10-, or 11-1H-pyrrolo[1,2-b][2]benzazapinyl. Typical fused heteroary groups include, but are not limited to 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, and 2-, 4-, 5-, 6-, or 7-benzothiazolyl.

A substituted heteroaryl is a heteroaryl group containing one or more substituents.

As used herein, the term "heterocyclyl" refers to a heterocyclic group that is saturated or partially saturated and is preferably a monocyclic or a polycyclic ring (in case of a polycyclic ring particularly a bicyclic, tricyclic or spirocyclic ring); and has 3 to 24, more preferably 4 to 16, most preferably 5 to 10 and most preferably 5 or 6 ring atoms; wherein one or more, preferably one to four, especially one or two ring atoms are a heteroatom (the remaining ring atoms therefore being carbon). The bonding ring (i.e. the ring connecting to the molecule) preferably has 4 to 12, especially 5 to 7 ring atoms. The term heterocyclyl excludes heteroaryl. The heterocyclic group can be attached at a heteroatom or a carbon atom. The heterocyclyl can include fused or bridged rings as well as spirocyclic rings. Examples of heterocycles include tetrahydrofuran (THF), dihydrofuran, 1, 4-dioxane, morpholine, 1,4-dithiane, piperazine, piperidine, 1,3-dioxolane, imidazolidine, imidazoline, pyrroline, pyrrolidine, tetrahydropyran, dihydropyran, oxathiolane, dithiolane, 1,3-dioxane, 1,3-dithiane, oxathiane, thiomorpholine, and the like.

A substituted heterocyclyl is a heterocyclyl group independently substituted by 1-4, such as one, or two, or three, or four substituents.

As used herein, the term "aryl" refers to an aromatic hydrocarbon group having 6-20 carbon atoms in the ring portion. Typically, aryl is monocyclic, bicyclic or tricyclic aryl having 6-20 carbon atoms. Furthermore, the term "aryl" as used herein, refers to an aromatic substituent which can be a single aromatic ring, or multiple aromatic rings that are fused together. Non-limiting examples include phenyl, naphthyl or tetrahydronaphthyl.

A substituted aryl is an aryl group substituted by 1-5 (such as one, or two, or three) substituents independently selected from the group consisting of hydroxyl, thiol, cyano, nitro, C₁-C₄-alkyl, C₁-C₄-alkenyl, C₁-C₄-alkynyl, C₁-C₄-alkoxy, C₁-C₄-thioalkyl, C₁-C₄-alkenyloxy, C₁-C₄-alkynyloxy, halogen, C₁-C₄-alkylcarbonyl, carboxy, C₁-C₄-alkoxycarbonyl, amino, C₁-C₄-alkylamino, di- C₁-C₄-alkylamino, C₁-C₄-alkylaminocarbonyl, di- C₁-C₄-alkylaminocarbonyl, C₁-C₄-alkylcarbonylamino, C₁-C₄-alkylcarbonyl(C₁-C₄-alkyl)amino, sulfonyl, sulfamoyl, alkylsulfamoyl, C₁-C₄-alkylaminosulfonyl where each of the afore-mentioned hydrocarbon groups (*e*.*g*., alkyl, alkenyl, alkynyl, alkoxy residues) may be further substituted by one or more residues independently selected at each occurrence from halogen, hydroxyl or C₁-C₄-alkoxy groups.

As used herein, the terms "about" and "substantially" indicate with respect to features such as endotherms, endothermic peak, exotherms, baseline shifts, etc., that their values can vary. With reference to X-ray diffraction peak positions, "about" or "substantially" means that typical peak position and intensity variability are taken into account. For example, one skilled in the art will appreciate that the peak positions (2θ) will show some inter-apparatus variability, typically as much as 0.2°. Occasionally, the variability could be higher than 0.2° depending on apparatus calibration differences. Further, one skilled in the art will appreciate that relative peak intensities will show inter-apparatus variability as well as variability due to degree of crystallinity, preferred orientation, prepared sample surface, and other factors known to those skilled in the art, and should be taken as qualitative measure only. For DSC, variation in the temperatures observed will depend upon the rate of temperature change as well as sample preparation technique and the particular instrument employed. Thus, the endotherm/melting point values reported herein relating to DSC/TGA thermograms can vary ± 5°C (and still be considered to be characteristic of the particular crystalline form described herein). When used in the context of other features, such as, for example, percent by weight (% by weight), reaction temperatures, the term "about" indicates a variance of ± 5%.

As used herein, "substantially pure phase," when used in reference to any crystalline form of the compound of Formula I, means a compound having a phase purity of greater than about 90% by weight, including greater than about 90, 91, 92, 93, 94, 95, 96, 97, 98, and about 99% by weight, and also including equal to about 100% by weight of the compound of Formula I, based on the weight of the compound on an anhydrous basis. The term "phase pure" or "phase purity" herein refers to phase homogeneity with respect to a particular solid state form of the compound of Formula I and does not necessarily imply a high degree of chemical purity absent an express statement to that effect. Phase purity may be determined according to methods known in the art, for example, using XRPD to do quantitative phase analysis using one or more approaches known in the art, for example, via an external standard method, direct comparisons of line (peak) characteristics which are attributed to different phases in a particular spectra, or via an internal standard method. However XRPD quantification of phase purity can be complicated by the presence of amorphous material. Accordingly, other methods that may be useful for determining phase purity include, for example, solid state NMR spectroscopy, Raman and/or infrared spectroscopy. One of skilled in the art would readily understand these methods and how to employ these additional (or alternative) methods for determining phase purity.

As used herein, the terms "salt" or "salts" refers to an acid addition or base addition salt of a compound for use in the method of the invention. "Salts" include in particular "pharmaceutically acceptable salts". The term "pharmaceutically acceptable salts" refers to salts that retain the biological effectiveness and properties of the compounds of this invention and, which typically are not biologically or otherwise undesirable. In many cases, the compounds for use in the method of the inventions are capable of forming acid and/or base salts by virtue of the presence of amino and/or carboxyl groups or groups similar thereto.

Pharmaceutically acceptable acid addition salts can be formed with inorganic acids and organic acids, *e.g.,* acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts for use in the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, *e.g.,* in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes hat can be incorporated into compounds (i.e. LTA4H inhibitor as described herein) include isotopes of hydrogen, carbon, nitrogen, oxygen, phosphorous, fluorine, and chlorine, such as ²H, ³H, ¹¹C, ¹³C, ¹⁴C, ¹⁵N, ¹⁸F ³¹P, ³²P, ³⁵S, ³⁶Cl, ¹²⁵I respectively. The invention includes various isotopically labeled compounds as defined herein, for example, those into which radioactive isotopes, such as ³H and ¹⁴C, or those into which non-radioactive isotopes, such as ²H and ¹³C are present. Such isotopically labeled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F or labeled compound may be particularly desirable for PET or SPECT studies. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Pharmaceutically acceptable solvates for use in the method of the invention include those wherein the solvent of crystallization may be isotopically substituted, *e*.*g*. D₂O, d₆-acetone, d₆-DMSO.

Compounds for use in the method of the invention, i.e. compounds of formula (I) that contain groups capable of acting as donors and/or acceptors for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the invention further provides co-crystals comprising a compound of formula (I) for use in the method of the invention.

As used herein, the term "administering" in relation to a compound, e.g., an LTA4H inhibitor (e.g. a compound of Formula I or a specific compound described herein), or another agent, is used to refer to delivery of that compound to a patient by any route.

As used herein, the term "pharmaceutically acceptable carrier" includes any and all solvents, dispersion media, coatings, surfactants, antioxidants, preservatives (*e*.*g*., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The term "a therapeutically effective amount" of a compound for use in the method of the invention refers to an amount of said compound that will elicit the biological or medical response of a subject, for example, reduction or inhibition of an enzyme or a protein activity, or ameliorate symptoms of HS, alleviate HS conditions, slow or delay disease progression of HS, or prevent HS. In one non-limiting embodiment, the term "a therapeutically effective amount" refers to the amount of the compound that, when administered to a subject, is effective to (1) at least partially alleviating, inhibiting, preventing and/or ameliorating HS condition. In another non-limiting embodiment, the term "a therapeutically effective amount" refers to the amount of the compound that, when administered to a cell, or a tissue, or a non-cellular biological material, or a medium, is effective to at least partially reducing or inhibiting the activity of LTA4H; or reducing or inhibiting the expression of LTA4H partially or completely.

As used herein, the term "subject" refers to an animal. Typically, the animal is a mammal. A subject also refers to for example, primates (*e*.*g*., humans, male or female), cows, sheep, goats, horses, dogs, cats, rabbits, rats, mice, fish, birds and the like. In certain embodiments, the subject is a primate. In yet other embodiments, the subject is a human. The term "subject" is used interchangeably with "patient" when it refers to human.

As used herein, the phrase "population of patients" is used to mean a group of patients. In some embodiments of the disclosed methods, the LTA4H inhibitor (e.g., compound of Formula I or a compound disclosed in WO2015/092740 or any compound described herein) is used to treat a population of HS patients.

As used herein, the phrases "has not been previously treated with a systemic treatment for HS" and "naive" refer to an HS patient who has not been previously treated with a systemic agent, e.g., methotrexate, cyclosporine, or with a biological agent (such IL-12 and IL-23 blocking agents such as ustekinumab and guselkumab or with a TNF-alpha inhibitors such as Infliximab, or with an IL-17 blocking agent such as secukinumab, ixekizumab and brodalumab) for HS. Systemic agents (i.e., agents given orally, by injection, etc.) differ from local agents (e.g., topicals and phototherapy) in that systemic agents have a systemic (whole body) effect when delivered to a patient. In some embodiments of the disclosed methods, regimens, uses, kits, and pharmaceutical compositions, the patient has not been previously administered a systemic treatment for HS.

As used herein, the phrase "has been previously treated with a systemic agent for HS" is used to mean a patient that has previously undergone HS treatment using a systemic agent. Such patients include those previously treated with biologics, such IL-12 and IL-23 blocking agents such as ustekinumab and guselkumab or with a TNF-alpha inhibitors such as Infliximab, or with an IL-17 blocking agent such as secukinumab, ixekizumab and brodalumab, and those previously treated with non-biologics, such as with a systemic immunosuppressant or immunomodulators (e.g. cyclosporine, methotrexate and cyclophosphamide), with systemic treatment including retinoids (such as isotretinoin), dapsone, metformin and oral zinc treatment. In some embodiments of the disclosure, the patient has been previously administered a systemic agent for HS. In some embodiments, the patient has been previously administered a systemic agent for HS (e.g., methotrexate, cyclosporine), but the patient has not been previously administered a systemic biological drug (i.e., a drug produced by a living organism, e.g., antibodies, receptor decoys, etc.) for HS (e.g., secukinumab, ustekinumab, ixekizumab, brodalumab, TNF alpha inhibitors (etanercept, adalimumab, remicade, etc.). In this case, the patient is referred to as "biological-naive." In some embodiments, the patient is biological-naive.

As used herein, "selecting" and "selected" in reference to a patient is used to mean that a particular patient is specifically chosen from a larger group of patients on the basis of (due to) the particular patient having a predetermined criteria. Similarly, "selectively treating" refers to providing treatment to a patient having a particular disease, where that patient is specifically chosen from a larger group of patients on the basis of the particular patient having a predetermined criterion. Similarly, "selectively administering" refers to administering a drug to a patient that is specifically chosen from a larger group of patients on the basis of (due to) the particular patient having a predetermined criterion. By selecting, selectively treating and selectively administering, it is meant that a patient is delivered a personalized therapy based on the patient's personal history (e.g., prior therapeutic interventions, e.g., prior treatment with biologics), biology (e.g., particular genetic markers), and/or manifestation (e.g., not fulfilling particular diagnostic criteria), rather than being delivered a standard treatment regimen based solely on the patient's membership in a larger group. Selecting, in reference to a method of treatment as used herein, does not refer to fortuitous treatment of a patient having a particular criterion, but rather refers to the deliberate choice to administer treatment to a patient based on the patient having a particular criterion. Thus, selective treatment/administration differs from standard treatment/administration, which delivers a particular drug to all patients having a particular disease, regardless of their personal history, manifestations of disease, and/or biology. In some embodiments, the patient is selected for treatment based on having HS.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development or progression of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e*.*g*., stabilization of a discernible symptom), physiologically, (*e*.*g*., stabilization of a physical parameter), or both. More specifically, the term "treating" the disease HS refers to treating the inflammatory lesions in HS patients (in numbers or quality or reducing their volume and size), and/or treating the abscesses and inflammatory nodules and/or draining fistulae in HS patients, and/or decreasing the amount of scarring and/or relieving the functional limitations associated with scarring. Treating the disease HS also refers to alleviating the pain, fatigue and/or itching associated with HS, reducing pus release and reducing the odor associated with pus release, and/or improving the quality of life and/or reducing the work impairment for HS patients.

As used herein, the term "prevention" refers delaying the onset or development or progression of the disease or disorder. More specifically, the term "preventing" the disease HS refers to preventing HS flares and or new lesions to appear; preventing scarring and preventing functional limitations associated with scarring and/or in particular preventing surgical interventions for HS.

As used herein, a subject is "in need of' a treatment if such subject would benefit biologically, medically or in quality of life from such treatment.

As used herein, the term "a," "an," "the" and similar terms used in the context of the present invention (especially in the context of the claims) are to be construed to cover both the singular and plural unless otherwise indicated herein or clearly contradicted by the context.

Any asymmetric atom (*e*.*g*., carbon or the like) of the compound(s) for use in the method of the invention can be present in racemic or enantiomerically enriched, for example the (R)-, (*S*)- or (*R,S*)- configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (*R*)- or (*S*)- configuration. Substituents at atoms with unsaturated double bonds may, if possible, be present in *cis-* (*Z*)- or *trans-* (*E*)*-* form.

Accordingly, a compound for use in the method of the present invention can be in the form of one of the possible isomers, rotamers, atropisomers, tautomers or mixtures thereof, for example, as substantially pure geometric (*cis* or *trans*) isomers, diastereomers, optical isomers (antipodes), racemates or mixtures thereof. For greater clarity, the term "possible isomers" shall not include positional isomers.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of final products or intermediates can be resolved into the optical antipodes by known methods, *e*.*g*., by separation of the diastereomeric salts thereof, obtained with an optically active acid or base, and liberating the optically active acidic or basic compound. In particular, a basic moiety may thus be employed to resolve the compounds of the present invention into their optical antipodes, *e*.*g*., by fractional crystallization of a salt formed with an optically active acid, *e*.*g*., tartaric acid, dibenzoyl tartaric acid, diacetyl tartaric acid, di-*O,O'-p*-toluoyl tartaric acid, mandelic acid, malic acid or camphor-10-sulfonic acid. Racemic products can also be resolved by chiral chromatography, *e*.*g*., high pressure liquid chromatography (HPLC) using a chiral stationary phase.

Furthermore, the compounds for use in the method of the invention, including their salts, can also be obtained in the form of their hydrates, or include other solvents used for their crystallization. The compounds for use in the method of the invention may by design form solvates with pharmaceutically acceptable solvents (including water); therefore, it is intended that the invention embrace the use of compounds as described herein, both solvated and unsolvated forms. The term "solvate" refers to a molecular complex of a compound for use in the method of the invention (including pharmaceutically acceptable salts thereof) with one or more solvent molecules. Such solvent molecules are those commonly used in the pharmaceutical art, which are known to be innocuous to the recipient, *e*.*g*., water, ethanol, and the like. The term "hydrate" refers to the complex where the solvent molecule is water.

The compounds for use in the method of the present invention, include salts, hydrates, solvates and polymorph thereof.

### LTA4H inhibitors

In one embodiment one, the present invention relates to a method of treating or preventing HS in a subject in need thereof, comprising administering to said subject a therapeutically effective amount of a LTA4H inhibitor.

In embodiment 1A, the present invention relates to a method of treating or preventing HS in a subject in need thereof, comprising administering to said subject a therapeutically effective amount of a LTA4H inhibitor as described in WO2014/164658.

In embodiment 1B, the present invention relates to a method of treating or preventing HS in a subject in need thereof, comprising administering to said subject a therapeutically effective amount of a LTA4H inhibitor which is: 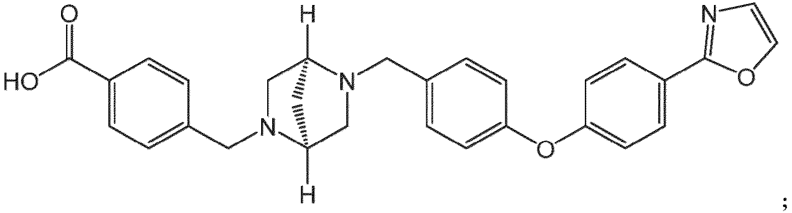 or a pharmaceutically acceptable salt thereof.

In embodiment 1C, the present invention relates to a method of treating or preventing HS in a subject in need thereof, comprising administering to said subject a therapeutically effective amount of a LTA4H inhibitor which is 4-(((1S,4S)-5-(4-(4-(oxazol-2-yl)phenoxy)benzyl)-2,5-diazabicyclo[2.2.1]heptan-2-yl)methyl)benzoic acid; or a pharmaceutically acceptable salt thereof.

In embodiment 1D, the present invention relates to a method of treating or preventing HS in a subject in need thereof, comprising administering to said subject a therapeutically effective amount of a LTA4H inhibitor which is Acebilustat, also known as CTX 4430.

In embodiment two, the present invention relates to a method of treating or preventing HS in a subject in need thereof, comprising administering to said subject a therapeutically effective amount of a compound of Formula (I), or a pharmaceutically acceptable salt thereof: 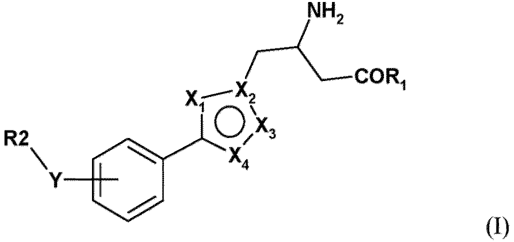 wherein,
R1 is OH or NH₂;
Y is O, S or CH₂;
X1, X2, X3 and X4 are N; or
X1, X2, X3 and X4 are selected from N, NH, C, CH and O with the proviso that at least two of
X1, X2, X3 or X4 are N or NH;
R2 is C₁-C₆ alkyl optionally substituted by phenyl; C₃-C₆ cycloalkyl; phenyl optionally being substituted by halogen, cyano, C₁-C₆ alkyl optionally substituted by halogen, C₁-C₆ alkoxy, or a 5-6 membered heteroaryl ring containing 1 to 3 heteroatoms selected from N, O and S; or a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen; or a pharmaceutically acceptable salt thereof.

Embodiment 2A relates to a method according to embodiment 2, comprising administering to the subject a therapeutically effective amount of a compound of formula (II) or a pharmaceutically acceptable salt thereof, 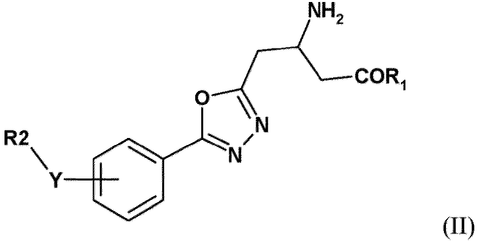 wherein the variables R1, R2 and Y have the meaning as defined in embodiment 1.

Embodiment 2B relates to a method according to embodiment 2, comprising administering to the subject a therapeutically effective amount of a compound of formula (III) or a pharmaceutically acceptable salt thereof, 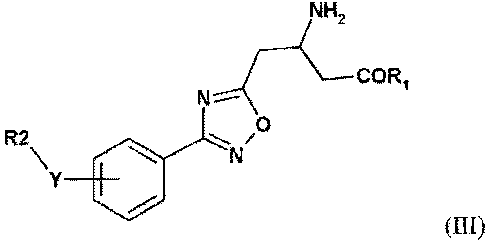 wherein the variables R1, R2 and Y have the meaning as defined in embodiment 1.

Embodiment 2C relates to a method of embodiment 2 comprising administering to the subject a therapeutically effective amount of a compound of formula (IV) or a pharmaceutically acceptable salt thereof, 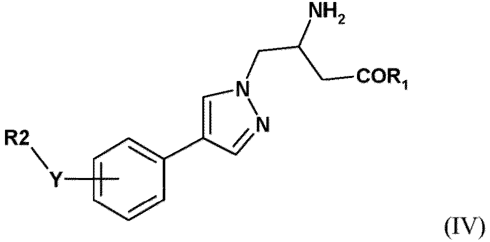 wherein the variables R1, R2 and Y have the meaning as defined in embodiment 1.

Embodiment 2D relates to a method in accordance to embodiment 2, comprising administering to the subject a therapeutically effective amount of a compound of formula (V) or a pharmaceutically acceptable salt thereof; 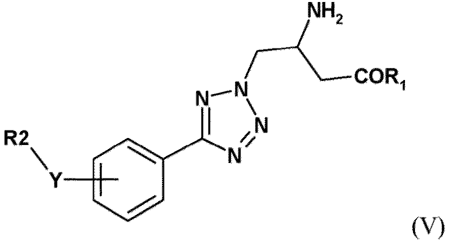 wherein the variables R1, R2 and Y have the meaning as defined in embodiment 1.

Embodiment 2E of the present invention relates to the method according to any one of embodiments 2 and 2A to 2D, comprising administering to the subject a therapeutically effective amount of a compound of formula (I), (II), (III), (IV) or (V), or a pharmaceutically acceptable salt thereof, wherein Y is O; and
R2 is phenyl optionally being substituted by halogen, cyano, C₁-C₆ alkyl optionally substituted by halogen, C₁-C₆ alkoxy, or a 5 - 6 membered heteroaryl ring containing 1 to 3 heteroatoms selected from N, O and S; or
R2 is a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen.

Embodiment 2F of the present invention relates to the method according to any one of embodiments 2and 2A to 2D, comprising administering to the subject a therapeutically effective amount of a compound of formula (I), (II), (III), (IV) or (V), or a pharmaceutically acceptable salt thereof, wherein Y is CH₂; and
R2 is phenyl optionally being substituted by halogen, cyano, C₁-C₆ alkyl optionally substituted by halogen, C₁-C₆ alkoxy, or a 5 - 6 membered heteroaryl ring containing 1 to 3 heteroatoms selected from N, O and S; or
R2 is a 5 - 10 membered mono- or bicyclic heteroaryl containing 1 to 4 heteroatoms selected from N, O and S, said heteroaryl being optionally substituted by cyano, halogen or C₁-C₆ alkyl optionally substituted by halogen.

Embodiment 2G of the present invention relates to a method according to any one of embodiments 2 and 2A to 2D, comprising administering to the subject a therapeutically effective amount of a compound of formula (I), (II), (III), (IV) or (V), or a pharmaceutically acceptable salt thereof; wherein Y is O; and
R2 is C₁-C₆ alkyl optionally substituted by phenyl; or C₃-C₆ cycloalkyl.

Embodiment 2H of the present invention relates to a method according to any one of embodiment 2 and 2A to 2D, comprising administering to the subject a therapeutically effective amount of a compound of formula (I), (II), (III), (IV) or (V), or a pharmaceutically acceptable salt thereof; wherein Y is CH₂; and
R2 is C₁-C₆ alkyl optionally substituted by phenyl; or C₃-C₆ cycloalkyl.

Embodiment 2I relates to a method according to any one of the embodiments 2 and 2A to 2H, comprising administering to the subject a therapeutically effective amount of a compound of formula (I), (II), (III), (IV) or (V), or a pharmaceutically acceptable salt thereof or a pharmaceutically acceptable salt thereof, wherein Y is attached in the para-position of the phenyl moiety.

Embodiment 2J relates to a method according to any one of the embodiments 2 and 2A to 2H, comprising administering to the subject a therapeutically effective amount of a compound of formula (I), (II), (III), (IV) or (V), or a pharmaceutically acceptable salt thereof, wherein Y is attached in the meta-position of the phenyl moiety.

Embodiment 2K relates to a method according to any one of the embodiments 2 and 2A to 2J, comprising administering to the subject a therapeutically effective amount of a compound of formula (I), (II), (III), (IV) or (V), or a pharmaceutically acceptable salt thereof, wherein R1 is OH.

Embodiment 2L relates to a method in accordance to any one of the embodiments 2 and 2A to 2K, comprising administering to the subject a therapeutically effective amount of a compound of formula (I), (II), (III), (IV) or (V), or a pharmaceutically acceptable salt thereof; wherein the amino group has the (*R*)-configuration.

Embodiment 2M relates to a method in accordance to any one of the embodiments 2 and 2A to 2K, comprising administering to the subject a therapeutically effective amount of a compound of formula (I), (II), (III), (IV) or (V), or a pharmaceutically acceptable salt thereof; wherein the amino group has the (*S*)-configuration.

Embodiment 2N relates to a method according to embodiment 2, comprising administering to the subject a therapeutically effective amount of a compound of formula (I) and/or a pharmaceutically acceptable salt thereof, wherein the compound is disclosed in WO2015/092740 [attorney docket number PAT056044-WO-PCT]; i.e. the compound is selected from:
(*R*)-3-amino-4-(5-(4-(benzo[*d*]thiazol-2-yloxy)phenyl)-2*H*-tetrazol-2-yl)butanoic acid;
(*R*)-3-amino-4-(5-(4-((5-chloropyridin-2-yl)oxy)phenyl)-2*H*-tetrazol-2-yl)butanoic acid;
(*R*)-3-amino-4-(5-(4-((5-chloro-3-fluoropyridin-2-yl)oxy)phenyl)-2*H*-tetrazol-2-yl)butanoic acid;
(*R*)-3-amino-4-(5-(4-(4-(oxazol-2-yl)-phenoxy)phenyl)-2*H*-tetrazol-2-yl)-butanoic acid;
(*R*)-3-amino-4-(5-(3-(4-chlorophenoxy)phenyl)-2*H*-tetrazol-2-yl)butanoic acid;
(*R*)-3-amino-4-(5-(4-(4-chlorophenoxy)-phenyl)-2*H*-tetrazol-2-yl)butanoic acid;
(*R*)-3-amino-4-(5-(4-(4-fluorophenoxy)-phenyl)-2*H-*tetrazol-2-yl)butanoic acid;
(*R*)-3-amino-4-(5-(4-(3-chloro-4-fluorophenoxy)pheny1)-2*H*-tetrazol-2-yl)butanoic acid;
(*R*)-3-amino-4-(5-(4-(p-tolyloxy)phenyl)-2*H*-tetrazol-2-yl)butanoic acid;
(*S*)-3-amino-4-(5-(3-phenoxyphenyl)-2*H*-tetrazol-2-yl)butanoic acid;
(*S*)-3-amino-4-(5-(4-(benzo[*d*]thiazol-2-yloxy)phenyl)-2*H*-tetrazol-2-yl)butanoic acid;
(*S*)-3-amino-4-(5-(4-(4-chlorophenoxy)-phenyl)-2*H*-tetrazol-2-yl)butanoic acid;
(*R*)-3-amino-4-(5-(3-phenethoxyphenyl)-2*H*-tetrazol-2-yl)butanoic acid;
(*R*)-3-amino-4-(5-(4-phenethoxyphenyl)-2*H*-tetrazol-2-yl)butanoic acid;
(*R*)-3-amino-4-(5-(4-(benzyloxy)phenyl)-2*H*-tetrazol-2-yl)butanoic acid;
(*R*)-3-amino-4-(5-(3-(benzyloxy)phenyl)-2*H*-tetrazol-2-yl)butanoic acid;
(*R*)-3-amino-4-(5-(4-butoxyphenyl)-27/-tetrazol-2-yl)butanoic acid;
(*R*)-3-amino-4-(5-(4-(pentyloxy)phenyl)-2*H*-tetrazol-2-yl)butanoic acid;
(*R*)-3-amino-4-(5-(3-((5-(trifluoromethyl)pyridin-2-yl)oxy)phenyl)-2*H*-tetrazol-2-yl)butanoic acid;
(*R*)-3-amino-4-(5-(4-((5-(trifluoromethyl)pyridin-2-yl)oxy)phenyl)-2*H*-tetrazol-2-yl)butanoic acid;
(*R*)-3-amino-4-(5-(3-(benzo[*d*]thiazol-2-yloxy)pheny1)-2*H*-tetrazol-2-yl)butanoicacid;
(*R*)-3-amino-4-(5-(3-(3,5-difluorophenoxy)phenyl)-2*H*-tetrazol-2-yl)butanoic acid;
(*S*)-3-amino-4-(5-(4-(*p*-tolyloxy)pheny1)-2*H*-tetrazol-2-yl)butanoic acid;
(*R*)-3-amino-4-(5-(4-(4-fluorophenoxy) phenyl)-1,3,4-oxadiazol-2-yl)butanoic acid;
(*R*)-3-amino-4-(5-(4-(4-chlorophenoxy) phenyl)-1,3,4-oxadiazol-2-yl)butanoic acid;
(*R*)-3-amino-4-(3-(4-(4-chlorophenoxy)phenyl)-1,2,4-oxadiazol-5-yl)butanoic acid;
(*R*)-3-amino-4-(3-(4-(4-chlorophenoxy)phenyl)-1,2,4-oxadiazol-5-yl)butanamide;
(*S*)-3-amino-4-(4-(4-(4-chlorophenoxy)phenyl)-1*H*-pyrazol-1-yl)butanoic acid; and
(*S*)-3-amino-4-(5-(4-((5-chloro-3-fluoropyridin-2-yl)oxy)phenyl)-2*H*-tetrazol-2-yl)butanoic acid.

In embodiment 3A, the invention relates to a method according to embodiment 2, comprising administering to the subject a therapeutically effective amount of a compound of formula (I) wherein the compound is (*S*)-3-amino-4-(5-(4-((5-chloro-3-fluoropyridin-2-yl)oxy)phenyl)-2*H-*tetrazol-2-yl)butanoic acid; or a pharmaceutically acceptable salt thereof.

In embodiment 3B, the invention relates to a method according to embodiment 2, comprising administering to the subject a therapeutically effective amount of a compound of formula (I) wherein the compound is (*R*)-3-amino-4-(5-(4-phenethoxyphenyl)-2*H*-tetrazol-2-yl)butanoic acid; or a pharmaceutically acceptable salt thereof.

In embodiment 3C, the invention relates to a method according to embodiment 2, comprising administering to the subject a therapeutically effective amount of a compound of formula (I) wherein the compound is (*R*)-3-amino-4-(5-(4-(4-chlorophenoxy)-pheny1)-2*H*-tetrazol-2-yl)butanoic acid; or a pharmaceutically acceptable salt thereof.

In embodiment 3D, the invention relates to a method according to embodiment 2, comprising administering to the subject a therapeutically effective amount of a compound of formula (I) wherein the compound is (*S*)-3-amino-4-(5-(4-(4-chlorophenoxy)-phenyl)-2*H*-tetrazol-2-yl)butanoic acid; or a pharmaceutically salt thereof.

The compounds of any one of Formulas (I) to (V) and the compounds according to any one of embodiments 2, 2A to 2N, 3 and 3A to 3C, for use in the method of the invention are disclosed in WO2015/092740, which is incorporated by reference herein.

In embodiment 3E, the invention relates to a method according to embodiment 2, comprising administering to the subject a therapeutically effective amount of a compound of formula (I) wherein the compound is crystalline form of (*S*)-3-amino-4-(5-(4-((5-chloro-3-fluoropyridin-2-yl)oxy)phenyl)-2*H*-tetrazol-2-yl)butanoic acid in its free form (i.e. non-salt form); or a pharmaceutically acceptable salt thereof.

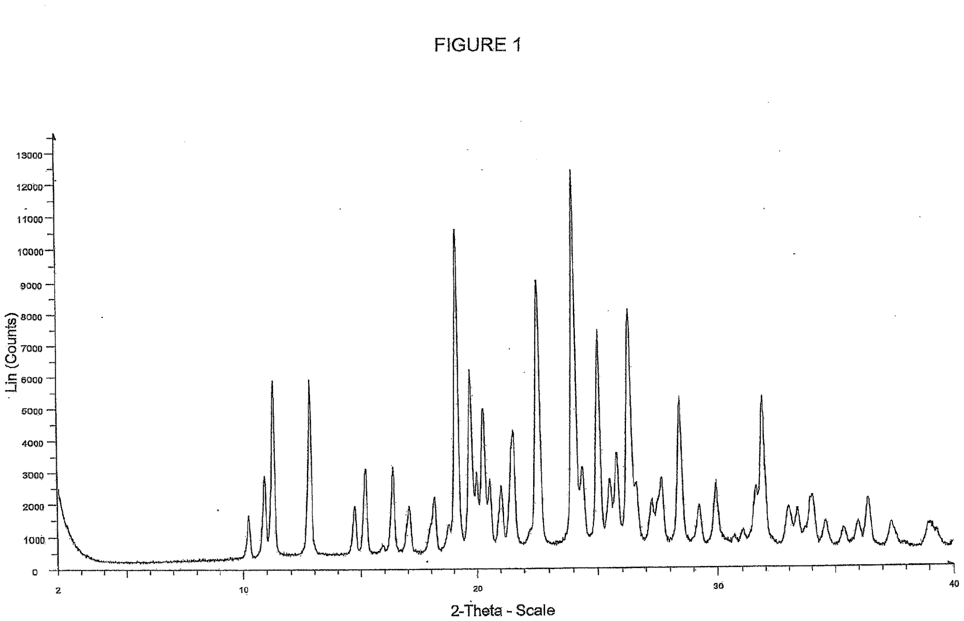

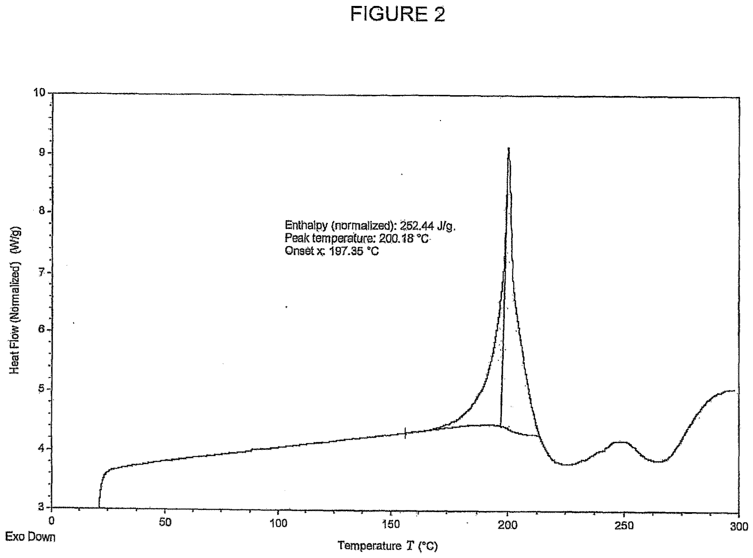

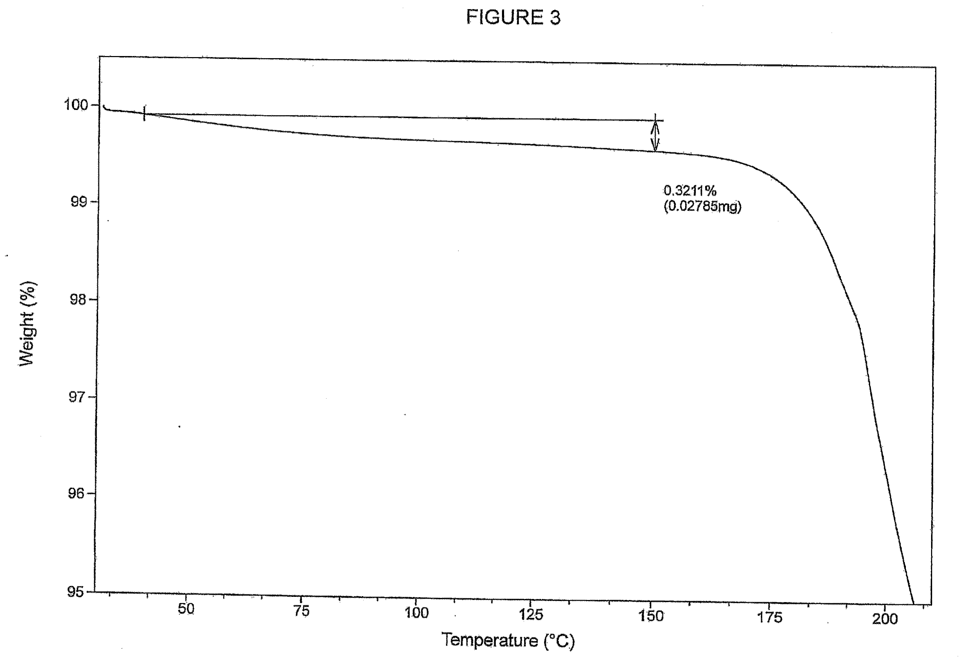

In embodiment 3F, the invention relates to the method according to embodiment 21, wherein the crystalline form is characterized by at least one of the following characteristics:
(i) an x-ray powder diffraction pattern comprising representative peaks in terms of 20 at 22.6 ± 0.2 °2Θ, 24.1 ± 0.2 °2Θ and 26.3 ± 0.2 °2Θ, measured at a temperature of about 25°C and an x-ray wavelength, λ, of 1.5418 Å;
(ii) an x-ray powder diffraction pattern comprising four or more 20 values selected from the group consisting of 11.3 ± 0.2 °2Θ, 12.8 ± 0.2 °2Θ, 15.2 ± 0.2 °2Θ, 19.7 ± 0.2 °2Θ, 20.0 ± 0.2 °2Θ, 20.3 ± 0.2 °2Θ, 21.0 ± 0.2 °2Θ, 22.6 ± 0.2 °2Θ, 24.1 ± 0.2 °2Θ, 24.4 ± 0.2 °2Θ, 25.1 ± 0.2 °2Θ, 26.3 ± 0.2 °2Θ, 28.5 ± 0.2 °2Θ, and 30.0 ± 0.2 °2Θ, measured at a temperature of about 25°C and an x-ray wavelength, λ, of 1.5418 Å;
(iii) an x-ray powder diffraction pattern comprising five or more 20 values selected from the group consisting of 11.3 ± 0.2 °2Θ, 12.8 ± 0.2 °2Θ, 15.2 ± 0.2 °2Θ, 19.7 ± 0.2 °2Θ, 20.0 ± 0.2 °2Θ, 20.3 ± 0.2 °2Θ, 21.0 ± 0.2 °2Θ, 22.6 ± 0.2 °2Θ, 24.1 ± 0.2 °2Θ, 24.4 ± 0.2 °2Θ, 25.1 ± 0.2 °2Θ, 26.3 ± 0.2 °2Θ, 28.5 ± 0.2 °2Θ, and 30.0 ± 0.2 °2Θ, measured at a temperature of about 25°C and an x-ray wavelength, λ, of 1.5418 Å;
(iv) an x-ray diffraction spectrum substantially the same as the x-ray powder diffraction spectrum shown in FIG. 1;
(v) a differential scanning calorimetry (DSC) thermogram substantially the same as that shown in shown in FIG. 2; and
(vi) a thermo gravimetric analysis (TGA) diagram substantially the same as that shown in shown in FIG. 3.

The crystalline form of embodiment 3D and 3E is disclosed in PAT058189-WO-PCT, which is PCT/CN2018/000278, filed on July 31st 2018, which is incorporated by reference herein.

### Methods of Treatment and Uses of LTA4H inhibitors for HS

The disclosed LTA4H inhibitors (i.e. compounds of any one of Formulae (I) to (V) and compounds according to any one of embodiments 1, 2, 2A to 2N, 3, and 3A to 3E, or a pharmaceutically acceptable salt thereof) may be used *in vitro, ex vivo,* or incorporated into pharmaceutical compositions and administered *in vivo* to treat HS patients (e.g., human patients).

HS is a chronic, inflammatory, scarring condition involving primarily the intertriginous skin of the axillary, inguinal, inframammary, genito-anal, and perineal areas of the body. It is also referred to as acne inversa. Three diagnostic criteria establish a diagnosis of HS: typical lesions (deep-seated painful nodules [blind] boils in early primary lesions, or abscesses, draining sinuses, bridged scars, and "tombstone" open comedones in secondary lesions); typical topography (axillae, groin, gentials, perineal and perianal regions, buttocks, and infra-and intermammary areas; and chronicity and recurrence (Margesson and Danby (2014) Best Practices and Res. Clin. Ob. And Gyn 28:1013-1027). The physical extent of HS can be classified using Hurley's clinical staging, shown below in **Table 2:**

**Table 2: Hurley's Stages of HS. Practically speaking, a patient having Hurley's stage III may have burned-out Stage III, but active Stage I or II lesions.**

| | |
|---|---|
| **Stage I** | Abscesses only (single or multiple) without sinus tracts and cicatrization (scarring) |
| **Stage II** | Abscesses (single or multiple) with tract formation or cicatrization, single or multiple widely separated lesions (e.g., > 10 cm apart) |
| **Stage III** | Diffuse or near diffuse involvement, or multiple interconnecting tracts or abscesses across entire area |

HS consists of follicular plugging, ductal rupture, and secondary inflammation. Patients first experience a plug in the follicular duct, which, over time leads to duct leak and horizontal rupture into the dermis. When repair of the folliculo-pilosebaceous (FPSB) fails, the follicular fragments stimulate three reactions that begin the HS disease course. The first is an inflammatory response, triggered by the innate immune system, causing purulence and tissue destruction, and leading to foreign body reactions and extensive scarring. The second reaction leads to epithelialized sinuses, which may evolve from stem cells derived from the FPSB unit that survive the destruction caused by the inflammatory response. Third, an invasive proliferative gelatinous mass is produced in most cases, consisting of a gel containing inflammatory cells, and, it is postulated, the precursors of the epithelialized elements described above. (See Margesson and Danby (2014). As used herein, the phrase "slowing HS disease progression" means decelerating the advancement rate of any of the aspects of the HS disease course described above, particularly the inflammatory response. In some embodiments of the disclosure, treatment with the LTA4H inhibitor slows HS disease progression.

Recurrence of HS in a patient includes the development of papules, pustules or inflammatory nodules, pain and itching, abscesses, draining, and any combination thereof. As used herein, "HS flare" (and the like) is defined as at least a 25% increase in abscesses and inflammatory nodule counts (AN), with a minimum increase of two ANs relative to a baseline.

In some embodiments of the disclosure, treatment according to the disclosed methods with the LTA4H inhibitors prevents HS flares, decreases the severity of HS flares, and/or decreases the frequency of HS flares. In some embodiments, when a population of HS patients is treated according to the disclosed methods, less than 5%, less than 10%, less than 15% or less than 20% experiences a flare during the treatment, for example during the first 16 weeks of treatment.

As used herein, the phrase "decreasing the severity of HS flares" and the like means reducing the intensity of an HS flare, e.g., reducing the number and/or size of abscesses and/or inflammatory nodules, reducing the strength of a particular flare component (e.g., reducing the number, size, thickness, etc. of abscesses and/or inflammatory nodules, reducing the extent of skin irritation (itching, pain) etc.), and/or reducing the amount of time a flare (or component thereof) persists.

As used herein, the phrase "decreasing the frequency of HS flares" and the like means reducing the incidence of HS flares, e.g., reducing the incidence of abscesses and/or inflammatory nodules. By decreasing the frequency of HS flares, a patient will experience fewer HS relapses. The incidence of flares may be assessed by monitoring a patient over time to determine if the prevalence of flares decreases.

As used herein, the phrase "preventing HS flares" means eliminating future HS flares and/or flare components.

The effectiveness of an HS treatment may be assessed using various known methods and tools that measure HS disease state and/or HS clinical response. Some examples include, e.g., Hurley's staging, severity assessment scoring system (SAHS), a Sartorius score, a modified Sartorius score, the HS physicians' global assessment (HS-PGA) score, a visual analog scale (VAS) or numeric rating scale (NRS) to rate skin related pain, the dermatology life quality index (DLQI), HS clinical response based on sum of abscesses and inflammatory nodules (HiSCR), simplified HiSCR, EuroQuol-5D (EQ5D), hospital anxiety and depression scale, healthcare resources utilization, Hidradenitis Suppurativa Severity Index (HSSI), Work productivity index (WPI), inflamed body surface area (BSA), Acne Inversa Severity Index (AISI) etc. (*see, e.g.,* Deckers and Prens (2016) Drugs 76:215-229; Sartorius et al. (2009) Br. J. Dermatol 161:831-39; Chiricozzi et al. (2015) Wounds 27(10):258-264). In some embodiments, the effectiveness of the method of the invention disclosed herein may be assessed by the HS physicians' global assessment (HS-PGA), severity assessment scoring score (SAHS), score numeric rating scale (NRS), the dermatology life quality index (DLQI), HS clinical response based on sum of abscesses and inflammatory nodules (HiSCR), and/or simplified HiSCR Preferably, the effectiveness of the HS treatment as disclosed herein may be assessed by HS clinical response based on sum of abscesses and inflammatory nodules (HiSCR), and/or simplified HiSCR.

In some embodiments, an HS patient achieves a HiSCR in response to HS treatment. In some embodiments, when a population of HS patients is treated according to the disclosed methods, at least 30%, at least 40%, at least 50%, at least 60% or at least 70% achieve a HiSCR by week 16 of treatment.

In other embodiments, the effectiveness of the HS treatment as disclosed herein can be measured by the difference between the responder rate in the treated patients (i.e. patient achieving a HiSCR response to the HS treatment with compound of the invention) and the responder rate in the placebo treated patients, by week 16 of treatment. In some embodiment, this difference in the responder rate as measured by HiSCR is at least 15%, at least 25%, at least 30% or at least 35%.

Preferred scoring systems for treatment response are the HiSCR, simplified HiSCR, NRS (especially NRS30), severity assessment scoring system (SAHS), HS-PGA, inflammatory lesion count (count of abscesses, inflammatory nodules, and/or draining fistulae), and the DLQI.

The Hidradenitis Suppurativa Clinical Response (HiSCR) is a measure of clinical response to HS treatment. A HiSCR response to treatment (compared to baseline) is as follows: 1) at least 50% reduction in abscesses and inflammatory nodules, and 2) no increase in the number of abscesses, and 3) no increase in the number of draining fistulae. As used herein the "simplified HiSCR" or "sHiSCR" refers to a modified HiSCR that does not include the abscess count versus baseline when assessing progression of lesions. In preferred embodiments, an HS patient achieves a simplified HiSCR in response to HS treatment. In some embodiments, when a population of HS patients is treated according to the disclosed methods, at least 40%, at least 50%, at least 60%, or at least 70% achieve a simplified HiSCR by week 16 of treatment.

Pain can be assessed using a numeric rating scale (NRS). In some embodiments, an HS patient achieves an improved NRS in response to HS treatment. NRS30 is defined as at least 30% reduction in pain and at least 1 unit reduction from baseline in Patient's Global Assessment (PGA) of Skin Pain from baseline in patients with a baselines score of 3 or higher. In some embodiments, an HS patient achieves an NRS30 in response to HS treatment. In some embodiments, when a population of HS patients is treated according to the disclosed methods, at least 25%, at least 30%, at least 40%, at least 50%, or at least 60% achieve an NRS30 by week 16 of treatment. In a preferred aspect of this embodiment, when a population of HS patients is treated according to the disclosed methods, at least 30% achieve an NRS30 by week 16 of treatment. In some embodiments, in response to treatment according to the claimed methods, the patient experiences rapid reduction in pain, as measured by VAS or NRS, as early as 1 week after initial dosing.

The severity assessment scoring system (SAHS) is described in JAMA Dermatol 2018, 154(3): 330-335, Hassam et al. The severity of HS can be assessed by the SAHS score, for which the following items are surveyed: number of involved regions (axilla left, axilla right, submammary left, submammary right, intermammary or chest, abdominal, mons pubis, groin left, groin right, genital, perianal or perineal, gluteal left, gluteal right, and others [eg, neck, retroauricular]), number of inflammatory and/or painful lesions other than fistula (ILOF), and number of fistula. These physician-rated items were completed by 2 patient-reported items: patients were asked for number of new boils or number of existing boils, which flared up during the past 4 weeks and to rate the current severity of pain (NRS) of the most symptomatic lesion in the course of their daily activities (e.g., sitting, moving, or working) on a numerical rating scale. The SAHS score is a composite score of all the collected information above. A mild case of HS is defined by a SAHS score of 4 or less. A moderate HS is defined by a SASH score of 5 to 8, and a severe case of HS is defined by SASH of 9 or higher.

In some embodiments, an HS patient achieves an improved SAHS score in response to HS treatment. In some embodiments, an HS patient achieves at least one point reduction from baseline in the SAHS score in response to HS treatment. In other embodiments, an HS patient achieves at least two points reduction or at least 3 points reduction from baseline in the SAHS score in response to HS treatment. Preferably, the SAHS score was at least 4 at baseline before treatment with a LTA4H inhibitor.

The DLQI is the most established dermatological life quality instrument. It consists of questions regarding the impact of the skin disease on feelings and different aspects of daily life activities during the last week. Each question is scored from 0 (not at all) to 3 (very much). A total of 30 points is the maximum score, where 0-1 is regarded as no effect, 2-5 small, 6-10 moderate, 11- 20 very large and 21-30 as extremely large effect on the patient's life. (*See* Finlay and Khan (1994) Clin Exp Dermatol 19:210-16). In some embodiments, an HS patient achieves an improved DLQI in response to HS treatment.

In some embodiments, in response to treatment according to the claimed methods, the patient experiences rapid reduction in CRP, as measured by standard CRP assay or a high sensitivity CRP (hsCRP) assay, as early as 1 week after initial dosing. As used herein, "C-reactive protein" and "CRP" refer to serum C-reactive protein, a plasma protein commonly used as an indicator of the acute phase response to inflammation. The level of CRP in plasma may be given in any concentration, e.g., mg/dl, nmol/L. Levels of CRP may be measured by a variety of standard assays, e.g., radial immunodiffusion, electroimmunoassay, immunoturbidimetry, ELISA, turbidimetric methods, fluorescence polarization immunoassay, and laser nephelometry. Testing for CRP may employ a standard CRP test or a high sensitivity CRP (hs-CRP) test (i.e., a high sensitivity test that is capable of measuring low levels of CRP in a sample using laser nephelometry). Kits for detecting levels of CRP may be purchased from various companies, e.g., Calbiotech, Inc, Cayman Chemical, Roche Diagnostics Corporation, Abazyme, DADE Behring, Abnova Corporation, Aniara Corporation, Bio-Quant Inc., Siemens Healthcare Diagnostics, etc.

The Sartorius HS score (also called the HS score, or HSS) is made by counting involved regions, nodules, and sinus tracts in an HS patient. (Sartorius et al. (2003) Br J Dermatol 149:211-13). The modified Sartorius HS score is a revision of the original version of the HSS by making minor simplifications which made it more practical to use, e.g., fewer specific lesions to include in the score, changes to the number of points given for each parameter, etc.( Sartorius et al. (2009) Br. J Dermatol. 161:831-839). In some embodiments, an HS patient achieves an improved modified Sartorius HS in response to HS treatment.

The HS physicians' global assessment (HS-PGA) is a 6-scale evaluating scale (scores range from 0-5) based on the number of HS lesions (i.e., abscesses, draining fistulas, inflammatory nodules, and noninflammatory nodules). (Kimball AB, Kerdel F, Adams D et al Adalimumab for the treatment of moderate to severe hidradenitis suppurativa: a parallel randomized trial. Ann Intern Med 2012; 157: 846-855). In some embodiments, an HS patient achieves an improved HS-PGA in response to HS treatment. In some embodiments, an HS patient achieves at least a 2 points reduction from baseline in the HS-PGA score in response to HS treatment. Preferably, the HS-PGA score was at least 3 at baseline before treatment with a LTA4H inhibitor.

In some embodiments, when a population of HS patients are treated according to the disclosed methods, at least 40%, at least 50%, at least 60%, at least 70%, at least 80% or at least 90% of patients who have responded to treatment by week 16 (e.g., patients achieving a HiSCR or simplified HiSCR by week 16) have sustained response after the end of treatment (for example 3 months or 6 months after the end of treatment, or 12 months after the end of treatment). In another aspect of this embodiment, at least 40% of patients or at least 50% of patients who have responded to treatment (e.g. patients achieving a HiSCR or simplified HiSCR, e.g. by week 16) have sustained response after the end of treatment (e.g. 3 months after the end of the treatment). Preferably at least 70% of patients who have responded to treatment (e.g., patients achieving a HiSCR or simplified HiSCR by e.g. week 16) have sustained response after the end of treatment (e.g. 3 months after the end of the treatment). As used herein the term "sustained" means that an outcome or goal (e.g., pain reduction, inflammation reduction) is substantially maintained for a given time.

The lesion-related itching can be assessed by a patient survey. The patient is asked to rate the lesion-related itching from 0 (no itching) to 10 (worse possible itching). In some embodiments, when a population of HS patients are treated according to the disclosed methods, the itching score improves by at least 2 points, preferably a least 3 points. Furthermore, when compared to the placebo group, a difference between the treated group and the placebo group is at least 1 point.

The odor caused by the draining of the lesion can be assessed by a patient survey. The patient is asked to rate the odor caused by the draining of the lesion from 1 (no odor), 2 (a little odor), 3 (moderate odor) to 4 (a lot of odor). In some embodiments, when a population of HS patients are treated according to the disclosed methods, the itching score improves by at least 1 point, preferably a least 2 points. Furthermore, when compared to the placebo group, a difference between the treated group and the placebo group is at least 1 point.

The impact on HS on the ability to complete work can be assessed by a patient survey. The patient is asked to rate how much HS impacts the ability to complete work from 1 (no at all), 2 (a little), 3 (moderately), 4 (a great deal) to 5 (unable to do any work). In some embodiments, when a population of HS patients are treated according to the disclosed methods, the itching score improves by at least 1 point, preferably a least 2 points. Furthermore, when compared to the placebo group, a difference between the treated group and the placebo group is at least 1 point.

The LTA4H inhibitors disclosed herein may be used as a pharmaceutical composition and dosage form when combined with a pharmaceutically acceptable carrier. Such a composition may contain, in addition to a LTA4H inhibitor, carriers, various diluents, fillers, salts, buffers, stabilizers, solubilizers, and other materials known in the art. The characteristics of the carrier will depend on the route of administration. The pharmaceutical composition for use in the method of the invention can be formulated for particular routes of administration such as oral administration, parenteral administration, and rectal administration, etc. In addition, the pharmaceutical compositions for use in the method of the present invention can be made up in a solid form (including without limitation capsules, tablets, pills, granules, powders or suppositories), or in a liquid form (including without limitation solutions, suspensions or emulsions). The pharmaceutical compositions for use in the method of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. Pharmaceutical compositions and dosage forms for use in the present invention may comprise one or more agents that reduce the rate by which the compound (i.e. LTA4H inhibitor described herein) as an active ingredient will decompose. Such agents, which are referred to herein as "stabilizers," include, but are not limited to, antioxidants such as ascorbic acid, pH buffers, or salt buffers, etc.

Typically, the pharmaceutical compositions for use in the invention are tablets or capsules comprising the active ingredient together with
a) fillers, e.g., lactose, dextrose, sucrose, mannitol, sorbitol, cellulose and/or glycine;
b) lubricants, e.g., silica, talcum, stearic acid, its magnesium or calcium salt (e.g. Magensium, stearate) and/or polyethyleneglycol;
c) binders, e.g., magnesium aluminum silicate, starch paste, gelatin, tragacanth, methylcellulose such as hydroxypropyl methylcellulose (HPMC or hypromellose), sodium carboxymethylcellulose and/or polyvinylpyrrolidone; if desired
d) disintegrants, e.g., crospovidone, starches, agar, alginic acid or its sodium salt, or effervescent mixtures;
e) glidant such as silica, colloidal anhydrous / colloidal silicon dioxide and/or
f) absorbents, colorants, flavors and sweeteners.

Tablets may be either film coated or enteric coated according to methods known in the art. Suitable compositions for oral administration include an effective amount of a compound of Formula I (or a compound according to any one of embodiments 1, 2, 2A to 2N, 3, and 3A to 3E) in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients, which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Suitable compositions for transdermal application include an effective amount of a compound for use in the method of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

In a preferred embodiment, suitable composition is for oral administration.

In preferred embodiments of the disclosed methods, uses and kits, LTA4H inhibitor (e.g. a compound according to any one of embodiments 1, 2, 2A to 2N, 3, and 3A to 3E, or a pharmaceutically acceptable salt thereof) is disposed in a pharmaceutical formulation, wherein said pharmaceutical formulation further comprises one or more fillers in an amount of 70-95% by weight, a desintegrant in an amount of 3-5% by weight and a binder in an amount of 2-3% by weight. In a particular aspect of this embodiment, the formulation further provides a lubricant in an amount of 1% by weight and a glidant in an amount on 0.5% by weight.

In some embodiments of the disclosed methods, uses and kits, the pharmaceutical formulation is in capsule or tablet form. In some embodiments of the disclosed methods, uses and kits, the pharmaceutical formulation is in a tablet form.

In some embodiments of the disclosed methods, uses and kits, the pharmaceutical formulation is in capsule form. In a specific aspect of this embodiment, the capsule fill comprises a compound according to any one of embodiments 1, 2, 2A to 2N, 3, and 3A to 3E, or a pharmaceutically acceptable salt thereof, a filler consisting of microcrystalline cellulose and mannitol wherein the amount of filler is 70-95% by weight of total capsule fill, crospovidone in an amount of 3-5% by weight of total capsule fill, hypromellose in an amount of 2-3% by weight of total capsule fill, magnesium stereate in an amount of about 1% by weight of total capsule fill, silica and colloidal silicon dioxide in an amount of about 0.5% by weight of total capsule fill.

The pharmaceutical compositions for use in the disclosed methods may also contain additional therapeutic agents for treatment of the particular targeted disorder. For example, a pharmaceutical composition may also include anti-inflammatory agents. Such additional factors and/or agents may be included in the pharmaceutical composition to produce a synergistic effect with the LTA4H inhibitor described herein, or to minimize side effects caused by the LTA4H inhibitor described herein.

Various therapies may be beneficially combined with the disclosed LTA4H inhibitors such as compounds of any one of Formulae (I) to (V) or a compound according to any one of embodiments 1, 2, 2A to 2N, 3, and 3A to 3E, or a pharmaceutically acceptable salt thereof, during treatment of HS.

Therefore, the LTA4H inhibitors of the present invention may be administered either simultaneously with, or before or after, one or more other therapeutic agent. The LTA4H inhibitors for use in the method of the invention may be administered separately, by the same or different route of administration, or together in the same pharmaceutical composition as the other agents.

In one embodiment, the invention pertains to the method of treating or preventing HS in a subject, comprising administering to the subject a product comprising a compound according to any one of formulae (I) to (V) (or a compound according to any one of embodiments 1, 2, 2A to 2N, 3, and 3A to 3E), or a pharmaceutically acceptable salt thereof, and at least one other therapeutic agent as a combined preparation for simultaneous, separate or sequential use in therapy. Optionally, the pharmaceutical composition for use in the method of the invention may comprise a pharmaceutically acceptable excipient, as described above.

Products provided as a combined preparation for use in the method of the invention, include a composition comprising the compound according to any one of formulae (I) to (V) (or a compound according to any one of embodiments 1, 2, 2A to 2N, 3, and 3A to 3E), or a pharmaceutically acceptable salt thereof, and the other therapeutic agent(s) together in the same pharmaceutical composition, or the compound according to any one of formulae (I) to (V) (or a compound according to any one of embodiments 1, 2, 2A to 2N, 3, and 3A to 3E), or a pharmaceutically acceptable salt thereof, and the other therapeutic agent(s) in separate form, *e.g.* in the form of a kit.

In one embodiment, the invention provides a kit for use in the method of the invention, comprising two or more separate pharmaceutical compositions, at least one of which contains a compound according to any one of formulae (I) to (V) (or a compound according to any one of embodiments 1, 2, 2A to 2N, 3, and 3A to 3E), or a pharmaceutically acceptable salt thereof. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the LTA4H inhibitor described herein and the other therapeutic agent may be manufactured and/or formulated by the same or different manufacturers. Moreover, the LTA4H inhibitors described herein and the other therapeutic may be brought together into a combination therapy: (i) prior to release of the combination product to physicians (e.g. in the case of a kit comprising the compound for use in the method of the invention and the other therapeutic agent); (ii) by the physician themselves (or under the guidance of the physician) shortly before administration; (iii) in the patient themselves, e.g. during sequential administration of the compound (LTA4H inhibitor) and the other therapeutic agent.

Accordingly, the invention provides the use of a compound according to any one of formulae (I) to (V) (or a compound according to any one of embodiments 1, 2, 2A to 2N, 3, and 3A to 3E), or a pharmaceutically acceptable salt thereof, for treating or preventing HS, wherein the medicament is prepared for administration with another therapeutic agent. The invention also provides the use of another therapeutic agent for treating or preventing HS, wherein the medicament is administered with a compound according to any one of formulae (I) to (V) (or a compound according to any one of embodiments 1, 2, 2A to 2N, 3, and 3A to 3E), or a pharmaceutically acceptable salt thereof.

The invention also provides a compound according to any one of formulae (I) to (V) (or a compound according to any one of embodiments 1, 2, 2A to 2N, 3, and 3A to 3E), or a pharmaceutically acceptable salt thereof, for use in a method of treating or preventing HS, wherein said compound is prepared for administration with another therapeutic agent. The invention also provides another therapeutic agent for use in a method of treating or preventing HS, wherein the other therapeutic agent is prepared for administration with a compound according to any one of formulae (I) to (V) (or a compound according to any one of embodiments 1, 2, 2A to 2N, 3, and 3A to 3E), or a pharmaceutically acceptable salt thereof.

The invention also provides a compound according to any one of formulae (I) to (V) (or a compound according to any one of embodiments 1, 2, 2A to 2N, 3, and 3A to 3E), or a pharmaceutically acceptable salt thereof, for use in a method of treating or preventing HS, wherein said compound is administered with another therapeutic agent. The invention also provides another therapeutic agent for use in a method of treating or preventing HS, wherein the other therapeutic agent is administered with a compound according to anyone of formulae (I) to (V) (or a compound according to any one of embodiments 1, 2, 2A to 2N, 3, and 3A to 3E), or a pharmaceutically acceptable salt thereof.

The invention also provides the use of a compound according to any one of formulae (I) to (V) (or a compound according to any one of embodiments 1, 2, 2A to 2N, 3, and 3A to 3E), or a pharmaceutically acceptable salt thereof, for treating and/or preventing HS in a patient in need of such treatment and/or prevention, wherein the patient has previously (e.g. within 24 hours) been treated with another therapeutic agent. The invention also provides the use of another therapeutic agent for treating or preventing HS in a patient in need thereof, wherein the patient has previously (e.g. within 24 hours) been treated with a compound according to any one of formulae (I) to (V) (or a compound according to any one of embodiments 1, 2, 2A to 2N, 3, and 3A to 3E), or a pharmaceutically acceptable salt thereof.

Such combined therapies include topical treatments (creams [non-steroidal or steroidal], washes, antiseptics,), systemic treatments (e.g., with biologicals, antibiotics, or chemical entities), antiseptics, photodynamic therapy, and surgical intervention (laser, draining or incision, excision).

Examples of oral antibiotics are tetracyclines and Rifampicin.

Non-limiting examples of topical HS agents for use with the disclosed LTA4H inhibitors, include benzoyl peroxide, topical steroid creams, topical antibiotics in the aminoglycoside group, such as clindamycin, gentamicin, and erythromycin, resorcinol cream, iodine scrubs, and chlorhexidine.

Non-limiting examples of HS agents used in systemic treatment for use with the disclosed LTA4H inhibitors, include IL-17 antagonists (ixekizumab, brodalumab, secukinumab CJM112), but as well IL17A /F antagonists such as bimekizumab or IL17C antagonists such as MOR106, tumor necrosis factor-alpha (TNF-alpha) blockers (such as Enbrel^{®} (etanercept), Humira^{®} (adalimumab), Remicade^{®} (infliximab) and Simponi^{®} (golimumab)), interleukin 12/23 blockers (such as Stelara^{®} (ustekinumab), tasocitinib, and briakinumab), IL-23 blockers (such as guselkumab, tildrakizumab and risankizumab) p19 inhibitors, PDE4 inhibitors such as apremilast or Otezla^{®}), complement pathway inhibitors, such as Factor B inhibitors (for example compounds disclosed in WO2015/009616, or LNP023 which is also known as 4-((2*S*,4*S*)-4-ethoxy-1-((5-methoxy-7-methyl)-1H indol-4-yl)methyl)piperidin-2-yl)benzoic acid), C5a inhibitors (IFX-001, CCX168 also known as Avacopan) , IL-1 antagonists (canakinumab, gevokizumab, rilonacept, anakinra, MaBp1 (XBiotech),), Inflammasome inhibitors such as NLRP3 and NLRP5 inhibitors, CXCR1/2 inhibitors, IL-18 antagonists, IL-6 antagonists, IL-36 antagonists, CD20 antagonists, CTLA4 antagonists, IL-8 antagonists, B-cell depletors (particularly CD20 antagonists, such as rituximab, as well as BAFF-R and CD40 antagonists such as Iscalimab (CFZ533)), IL-21 antagonists, IL-22 antagonist, VEGF antagonists, CXCL antagonists, MMP antagonists, and defensin antagonists (e.g., receptor decoys, antagonistic antibodies, etc.), as well as broad spectrum oral JAK inhibitors or more specific TYK2 or JAK 1, JAK2 or JAK 3 inhibitors.

Additional HS agents for use in combination with the disclosed LTA4H inhibitors, during treatment of HS include retinoids, such as Acitretin (e.g., Soriatane ^{®}) and isotretinoin, immune system suppressants (e.g., rapamycin, T-cell blockers [e.g., Amevive^{®} (alefacept) and Raptiva^{®} [efalizumab]) cyclosporine, methotrexate, mycophenolate mofetil, mycophenolic acid, leflunomide, tacrolimus, etc.), hydroxyurea (e.g., Hydrea^{®}), sulfasalazine, 6-thioguanine, fumarates (e.g, dimethylfumarate and fumaric acid esters), azathioprine, colchicine, alitretinoin, steroids, corticosteroids, certolizumab, mometasone, rosiglitazone, pioglitazone, botulinum toxin, triamcinolone, IFX-1 (InflaRx), LY-3041658 (Eli Lilly), TE-2232 (Immunwork), NSAIDs, COX inhibitors, prescription narcotics, ketoprofen, codeine, gabapentin, pregabalin gentanyl, antibiotics (topical, oral, IV) (e.g., clindamycin, rifampin, tetracycline, sarecycline, doxycycline, minocycline, lymecycline, trimethoprim-sulfamethoxazole, erythromycin, ceftriaxone, moxifloxacin, metronidazole, separately or as combinations), corticosteroid (injectable or oral), antiandrogen/hormonal therapy (oral contraceptives, spironolactone, finasteride, dutasteride, progesterone IUD, cyproterone acetate, ethinyloestradiol, gestodene, norgestimate, desogestrel, drospirenone, spironolactone), Triamcinolone Acetonide, MEDI8968, hydroxychloroquine, dapsone, metformin, adapalene, azelaic acid and zinc.

Preferred combinations for used in the disclosed kits, methods, and uses include the PDE4i and JAKi, as well as antibiotics (all oral).

Examples of JAK inhibitors for use in combination are BMS986165, INCB054707, Ruxolitinib, Abrocitinib, Tofacitinib and Baricitinib. Other examples of JAK inhibitors are compounds disclosed in WO2017/089985, WO2018/055550 and WO2018/055551.

A skilled artisan will be able to discern the appropriate dosages of the above HS agents for co-delivery with the disclosed LTA4H inhibitors.

A LTA4H inhibitor, e.g. a compound according to any one of embodiments 1, 2, 2A to 2N, 3, and 3A to 3E, or a pharmaceutically acceptable salt thereof, is conveniently administered orally. The duration of the oral therapy using a pharmaceutical composition of the present disclosure will vary, depending on the severity of the disease being treated and the condition and personal response of each individual patient. The health care provider will decide on the appropriate duration of oral therapy and the timing of administration of the therapy, using the pharmaceutical composition of the present disclosure. In some embodiments, the patient is treated for HS according to the claimed methods for at least 16 weeks, at least 24 weeks, at least 36 weeks, at least 48 weeks, at least 52 weeks. In some embodiments, the patient is treated for HS in a chronic use.

In one embodiment, the pharmaceutical composition of the present invention for use in the prevention or the treatment of HS, can be in unit dosage of about 1-500 mg of active ingredient(s) for a subject of about 50-70 kg, or about 1-250 mg or about 1-150 mg or about 1-100 mg, or about 1-50 mg of active ingredients. The therapeutically effective dosage of a compound, the pharmaceutical composition is dependent on the species of the subject, the body weight, age and individual condition, the severity of the contrast-induced nephropathy disorder. A physician, clinician or veterinarian of ordinary skill can readily determine the effective amount of each of the active ingredients necessary to prevent, treat or inhibit the progress of the disorder or disease.

Preferred formulation is a capsule or a tablet composition comprising from about 1 mg to about 160mg of a LTA4H inhibitor or of a compound according to any one of embodiments 1, 2, 2A to 2N, 3, and 3A to 3E, or a pharmaceutically acceptable salt thereof, and one or more excipients independently selected from fillers, desintegrants, binders, and optionally lubricant and glidant. In a preferred embodiment, the capsule or tablet composition comprises from about 5 mg to about 80mg of a LTA4H inhibitor or of a compound according to any one of embodiments 1, 2, 2A to 2N, 3, and 3A to 3E, or a pharmaceutically acceptable salt thereof, and and one or more excipients independently selected from fillers, desintegrants, binders, and optionally lubricant and glidant. In yet another embodiment, the capsule or tablet composition comprises about 1mg, about 5mg, about 10mg, about 20mg, about 30mg, about 40mg and about 50mg of a LTA4H inhibitor or of a compound according to any one of embodiments 1, 2, 2A to 2N, 3, and 3A to 3E, or a pharmaceutically acceptable salt thereof, and one or more excipients independently selected from fillers, desintegrants, binders, and optionally lubricant and glidant.

Disclosed herein are methods of treating hidradenitis suppurativa (HS), comprising orally administering to a patient in need thereof a dose of about 1mg to about 160 mg, or about 10mg to about 100mg, or about 20 to about 60mg of a LTA4H inhibitor (i.e. a compound according to any one of embodiments 1, 2, 2A to 2N, 3, and 3A to 3E, or a pharmaceutically acceptable salt thereof), daily. Said doses can be administered to the patient either with a once a day dosing regimen or twice a day dosing regimen. In another embodiment, the method comprises orally administering to a patient in need thereof a dose of about 10mg to about 30 mg of a LTA4H inhibitor (i.e. a compound according to any one of embodiments 1, 2, 2A to 2N, 3, and 3A to 3E, or a pharmaceutically acceptable salt thereof), twice a day (BID). In a preferred aspect of this embodiment, the method comprises orally administering a dose of about 20 mg of LTA4H inhibitor (i.e. a compound according to any one of embodiments 1, 2, 2A to 2N, 3, and 3A to 3E, or a pharmaceutically acceptable salt thereof), to said patient twice a day (BID).

Disclosed herein are methods of treating hidradenitis suppurativa (HS), comprising orally administering to a patient in need thereof a daily dose of about 1mg to about 160 mg, or about 10mg to about 100mg, or about 20 to about 60mg of a LTA4H inhibitor, wherein the LTA4H inhibitor is (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof. In a preferred aspect of this embodiment, the method comprises orally administering a dose of about 10mg to about 30 mg of (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof, to said patient twice a day. In a most preferred aspect of this embodiment, the method comprises orally administering a dose of about 20mg of (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof, to said patient twice a day.

Disclosed herein are methods of treating hidradenitis suppurativa (HS), comprising orally administering to a patient in need thereof a daily dose of about 1 mg to about 160 mg, or about 10mg to about 100mg, or about 20mg to about 60mg of a LTA4H inhibitor, wherein the LTA4H inhibitor is a crystalline form of (*S*)-3-amino-4-(5-(4-((5-chloro-3-fluoropyridin-2-yl)oxy)phenyl)-2*H*-tetrazol-2-yl)butanoic acid in its free form, as described herein. In a preferred aspect of this embodiment, the method comprises orally administering a dose of about 10mg to about 30 mg of a crystalline form of (*S*)-3-amino-4-(5-(4-((5-chloro-3-fluoropyridin-2-yl)oxy)phenyl)-2*H*-tetrazol-2-yl)butanoic acid, as described herein, to said patient twice a day. In a most preferred aspect of this embodiment, the method comprises orally administering a dose of about 20mg of a crystalline form of (*S*)-3-amino-4-(5-(4-((5-chloro-3-fluoropyridin-2-yl)oxy)phenyl)-2*H*-tetrazol-2-yl)butanoic acid, as described herein, to said patient twice a day.

Disclosed herein is a LTA4H inhibitor (i.e. a compound according to any one of embodiments 1, 2, 2A to 2N, 3, and 3A to 3E, or a pharmaceutically acceptable salt thereof), for use in the treatment of hidradenitis suppurativa (HS), wherein the LTA4H inhibitor is administered orally to a patient in need thereof in a daily dose of about 1mg to about 160mg, or about 4mg to about 100mg, or about 10mg to about 100mg, or about 20mg to about 60mg or about 5mg to about 80mg (e.g. about 20mg, about 30mg, about 40mg or about 80mg). In a preferred aspect of this embodiment, LTA4H inhibitor (e.g. a compound according to any one of embodiments 1, 2, 2A to 2N, 3, and 3A to 3E, or a pharmaceutically acceptable salt thereof) is administered orally to a patient in need thereof in a dose of about 5mg QD to about 40mg BID. In yet another preferred aspect of this embodiment, LTA4H inhibitor (e.g. a compound according to any one of embodiments 1, 2, 2A to 2N, 3, and 3A to 3E, or a pharmaceutically acceptable salt thereof) is administered orally to a patient in need thereof in a dose of about 10mg to about 30mg twice a day (e.g. about 10mg BID, about 15mg BID, preferably about 20mg BID). In yet a most preferred aspect of this embodiment, LTA4H inhibitor (e.g. a compound according to any one of embodiments 1, 2, 2A to 2N, 3, and 3A to 3E, or a pharmaceutically acceptable salt thereof) is administered orally to a patient in need thereof in a dose of about 20 mg twice a day.

Disclosed herein is a LTA4H inhibitor which is (*S*)-3-amino-4-(5-(4-((5-chloro-3-fluoropyridin-2-yl)oxy)phenyl)-2*H*-tetrazol-2-yl)butanoic acid or a pharmaceutically acceptable salt thereof, for use in the treatment of hidradenitis suppurativa (HS), wherein the LTA4H is administered orally to a patient in need thereof a daily dose of about 1mg to about 160mg, or about 4mg to about 100mg, or about 10mg to about 100mg, or about 20mg to about 60mg or about 5mg to about 80mg (e.g. about 20mg, about 30mg, about 40mg or about 80mg). In a preferred aspect of this embodiment, (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof, is administered orally to a patient in need thereof in a dose of about 5mg QD to about 40mg BID. In yet another preferred aspect of this embodiment, (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof, is administered orally to a patient in need thereof in a dose of about 10mg to about 30mg twice a day (e.g. about 10mg BID, about 15mg BID, preferably about 20mg BID). In yet a most preferred embodiment, (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof, is administered orally to a patient in need thereof in a dose of about 20mg twice a day.

Disclosed herein is a LTA4H inhibitor which is a crystalline form of (*S*)-3-amino-4-(5-(4-((5-chloro-3-fluoropyridin-2-yl)oxy)phenyl)-2*H*-tetrazol-2-yl)butanoic acid in its free form as described herein, for use in the treatment of hidradenitis suppurativa (HS), wherein the LTA4H is administered orally to a patient in need thereof a daily dose of about 1mg to about 160mg, or about 4mg to about 100mg, or about 10mg to about 100mg, or about 20mg to about 60mg or about 5mg to about 80mg (e.g. about 20mg, about 30mg, about 40mg or about 80mg). In a preferred aspect of this embodiment, the crystalline form of (*S*)-3-amino-4-(5-(4-((5-chloro-3-fluoropyridin-2-yl)oxy)phenyl)-2*H*-tetrazol-2-yl)butanoic acid in its free form as described herein, is administered orally to a patient in need thereof in a dose of about 10mg QD to about 40mg BID. In yet another preferred aspect of this embodiment, the crystalline form of (S)-3-amino-4-(5-(4-((5-chloro-3-fluoropyridin-2-yl)oxy)phenyl)-2*H*-tetrazol-2-yl)butanoic acid in its free form as described herein, is administered orally to a patient in need thereof in a dose of about 10mg to about 30mg twice a day (e.g. about 10mg BID, about 15mg BID, preferably about 20mg BID). In yet a most preferred embodiment, the crystalline form of (*S*)-3-amino-4-(5-(4-((5-chloro-3-fluoropyridin-2-yl)oxy)phenyl)-2*H*-tetrazol-2-yl)butanoic acid in its free form as described herein, is administered orally to a patient in need thereof in a dose of about 20mg twice a day.

In preferred embodiments of the disclosed methods, uses and kits, the dose of the LTA4H inhibitor is about 10mg to about 30mg BID or about 20 mg BID.

In preferred embodiments of the disclosed methods, uses and kits, the patient achieves a sustained response after one year of treatment, as measured by (simplified) Hidradenitis Suppurativa Clinical Response (HiSCR), Numerical Rating Scale (NRS), Hidradenitis Suppurativa - Physician Global Assessment (HS-PGA), or Dermatology Life Quality Index (DLQI).

In preferred embodiments of the disclosed methods, uses and kits, prior to treatment with a LTA4H inhibitor as disclosed herein, the patient has been previously treated with a systemic agent for HS. In preferred embodiments of the disclosed methods, uses and kits, the systemic agent is selected from the group consisting of a topical treatment, an antibiotic, an immune system suppressant, a TNF-alpha inhibitor, an IL-1 antagonist, and combinations thereof.

In some embodiments of the disclosed methods, uses and kits, prior to treatment with a LTA4H as described herein, the patient has not been previously treated with a systemic agent or a topical treatment for HS.

In one embodiments of the disclosed methods, uses and kits, the LTA4H inhibitor as described herein (e.g. a compound according to any one of embodiments 1, 2, 2A to 2N, 3, and 3A to 3E, or a pharmaceutically acceptable salt thereof), is administered in combination with at least one of an antibiotic, a JAK inhibitor, a TYK2 inhibitor a PDE4 inhibitor or an immunosuppressant.

In preferred embodiments of the disclosed methods, uses and kits, the dose of the LTA4H inhibitor as described herein (e.g. a compound according to any one of embodiments 1, 2, 2A to 2N, 3, and 3A to 3E, or a pharmaceutically acceptable salt thereof), is about 10mg to about 30mg BID. In other preferred embodiments of the disclosed methods, uses and kits, the dose of the LTA4H inhibitor (e.g. a compound according to any one of embodiments 1, 2, 2A to 2N, 3, and 3A to 3E, or a pharmaceutically acceptable salt thereof), is about 20 mg.

In preferred embodiments of the disclosed methods, uses and kits, the patient has moderate to severe HS.

As used herein, the phrase "moderate to severe" refers to HS disease in which patients have ≥3 active, inflammatory lesions [i.e., deep inflammatory lesions such as abscesses and/or inflammatory nodules], no more than 10 fistulae and at least two anatomical areas need to be involved in HS lesions.

In preferred embodiments of the disclosed methods, uses and kits, the patient is an adult.

In some embodiments of the disclosed methods, uses and kits, the HS patient is an adult with moderate to severe HS disease.

In some embodiments of the disclosed methods, uses and kits, the patient is an adolescent patient (≥ 12 years of age). In some embodiments, the patient is an adolescent patient having moderate to severe HS.

In some embodiments of the disclosed methods, uses and kits, the patient has been diagnosed with HS for at least one year.

In some embodiments of the disclosed methods, uses and kits, the patient does not have extensive scarring as a result of HS (i.e., < 20 fistulas, draining or not draining or <10 fistulas).

In some embodiments of the disclosed methods, uses and kits, the patient previously had an inadequate response to conventional systemic HS therapy.

In preferred embodiments of the disclosed methods, uses and kits, prior to treatment with a LTA4H inhibitor as described herein (e.g. a compound according to any one of embodiments 1, 2, 2A to 2N, 3, and 3A to 3E, or a pharmaceutically acceptable salt thereof), the patient has an HS-PGA score of ≥3.

In preferred embodiments of the disclosed methods, uses and kits, the patient achieves a (simplified) HiSCR by week 16 of treatment.

In preferred embodiments of the disclosed methods, uses and kits, the patient achieves an NRS30 by week 16 of treatment.

In preferred embodiments of the disclosed methods, uses and kits, the patient has a reduction in HS flares by week 16 of treatment.

In preferred embodiments of the disclosed methods, uses and kits, the patient achieves a reduction of ≤ 6 as measured by the DLQI by week 16 of treatment.

In preferred embodiments, when the disclosed methods, uses or kits are used to treat a population of patients with moderate to severe HS, at least 40% of said patients achieve a simplified HiSCR by week 16 of treatment in response to said administering step.

In another preferred embodiment, when the disclosed methods, uses or kits are used to treat a population of patients with moderate to severe HS, the difference between the responder rate (e.g. patient achieving a HiSCR response to the HS treatment) and the responder rate in the placebo treated patients, by week 16 of treatment is at least 15%, at least 25%, or at least 30%.

In preferred embodiments of the disclosed methods, uses and kits, the patient has a reduction in modified Sartorius score, e.g. by 16 weeks of treatment.

In preferred embodiments of the disclosed methods, uses and kits, the patient has an improvement in DLQI, e.g. by 16 weeks of treatment.

In preferred embodiments, when the disclosed methods, uses or kits are used to treat a population of patients with moderate to severe HS, at least 25% (and preferably at least 30%) of said patients achieve an NRS30 response, e.g. by week 16 of treatment in response to said administering step.

In preferred embodiments, when the disclosed methods, uses or kits are used to treat a population of patients with moderate to severe HS, less than 15% of said patients experience an HS flare during the treatment in response to said administering step (for example during 16 weeks of treatment).

In preferred embodiments of the disclosed methods, uses and kits, the patient is additionally treated with at least one topical medication and at least one antiseptic in combination with a LTA4H inhibitor as described herein.

In preferred embodiments of the disclosed methods, uses and kits, the patient is treated with a LTA4H inhibitor as described herein for at least 16 weeks, at least 24 weeks, at least 36 weeks, at least 48 weeks or at least 52 weeks. Most preferably, the patient is treated for at least 16 weeks.

In preferred embodiments of the disclosed methods, uses and kits, the patient has a rapid reduction in pain, as measured by VAS or NRS, as early as one week after the first dose of the LTA4H inhibitor thereof.

In preferred embodiments of the disclosed methods, uses and kits, the patient has a rapid reduction in CRP, as measured using a standard CRP assay, as early as one week after the first dose of the IL-17 antibody or antigen binding fragment thereof.

In preferred embodiments of the disclosed methods, uses and kits, the LTA4H inhibitor is (*S*)-3-amino-4-(5-(4-((5-chloro-3-fluoropyridin-2-yl)oxy)phenyl)-2*H*-tetrazol-2-yl)butanoic acid or a pharmaceutically acceptable salt thereof.

In preferred embodiments of the disclosure, the LTA4H inhibitor is a crystalline form of (*S*)-3-amino-4-(5-(4-((5-chloro-3-fluoropyridin-2-yl)oxy)phenyl)-2*H*-tetrazol-2-yl)butanoic acid in its free form (herein designated as Form B).

In preferred embodiments the LTA4H inhibitor is Form B as described herein in a substantially pure phase.

Aspects, advantageous features and preferred embodiments of the present invention which are summarized in the following items, respectively alone or in combination, contribute to solving the object of the invention:
Item 1. A method of treating or preventing hidradenitis suppurativa (HS), comprising administering to a subject in need thereof a therapeutically effective amount of a LTA4H inhibitor.
Item 2. The method according to item 1, wherein the LTA4H inhibitor is a compound of Formula (I), or a pharmaceutically acceptable salt thereof: 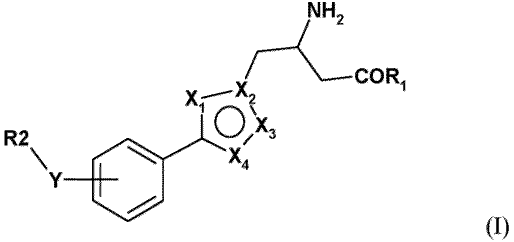 wherein,
   R1 is OH or NH₂;
   Y is O, S or CH₂;
   X1, X2, X3 and X4 are N; or
   X1, X2, X3 and X4 are selected from N, NH, C, CH and O with the proviso that at least two of
   X1, X2, X3 or X4 are N or NH;
   R2 is C₁-C₆ alkyl optionally substituted by phenyl; C₃-C₆ cycloalkyl; phenyl optionally being substituted by halogen, cyano, C₁-C₆ alkyl optionally substituted by halogen, C₁-C₆ alkoxy, or a 5-6 membered heteroaryl ring containing 1 to 3 heteroatoms selected from N, O and S; or a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen; or a pharmaceutically acceptable salt thereof.
Item 3. The method according of item 1 or 2, wherein the LTA4H inhibitor is (S)-3-amino-4-(5-(4-((5-chloro-3-fluoropyridin-2-yl)oxy)phenyl)-2*H*-tetrazol-2-yl)butanoic acid, or a pharmaceutically acceptable salt thereof.
Item 4. The method according to any of the above items, wherein the LTA4H inhibitor is disposed in a pharmaceutical formulation, wherein said pharmaceutical formulation comprises one or more pharmaceutically acceptable carriers, each of which is independently selected from a filler, a lubricant, a binder, a desintegrant and a glidant.
Item 5. The method according to item 4, wherein the pharmaceutical formulation is in tablet or capsule form.
Item 6. The method according to any of the above items, wherein the LTA4H inhibitor is administered in a daily dose of about 10mg to about 100 mg.
Item 7. The method according to any of the above items, wherein the LTA4H inhibitor is administered in combination with one or more therapeutic agents.
Item 8. The method according to any one of items 1 to 6, wherein the patient is additionally treated with at least one topical medication and at least one antiseptic in combination with the LTA4H inhibitor.
Item 9. The method according to any of the items 1 to 8, wherein, prior to treatment with the LTA4H inhibitor, the patient has not been previously treated with a systemic agent or a topical treatment for HS.
Item 10. The method according to any of the above items, wherein the patient is selected according to one of the following criteria:
   a) the patient has moderate to severe HS;
   b) prior to treatment with the LTA4H inhibitor, the patient has an HS-PGA score of ≥3;
   c) prior to treatment with the LTA4H inhibitor, the patient has at least 3 inflammatory lesions; or
   d) prior to treatment with the LTA4H inhibitor, the patient does not have extensive scarring (<10 fistulas) as a result of HS.
Item 11. The method according to any of the above items, wherein said patient achieves at least one of the following (e.g by week 16 of treatment):
   a) a simplified HiSCR;
   b) a reduction in HS flares;
   c) a NRS30;
   d) a reduction of ≤ 6 as measured by the DLQI; and/or
   e) an improvement in DLQI.
Item 12. The method according to any of the above items, wherein, when said method is used to treat a population of patients with moderate to severe HS, at least 40% of said patients achieve a simplified HiSCR, e.g. by week 16 of treatment.
Item 13. The method according to any of items 1 to 11, wherein, when said method is used to treat a population of patients with moderate to severe HS, or at least 25% of said patients achieve an NRS30 response, e.g. by week 16 of treatment.
Item 14. The method according to any of the items 1 to 11, wherein, when said method is used to treat a population of patients with moderate to severe HS, less than 15% of said patients experience an HS flare during the treatment, for example, during 16 weeks of treatment.
Item 15. The method according to any of the above items, wherein the patient is treated with the LTA4H inhibitor for at least 16 weeks.
Item 16. The method according to any of the items 1 to 11, wherein the patient has at least one of the followings as early as one week after the first dose of the LTA4H inhibitor:
   a) a rapid reduction in pain, as measured by VAS or NRS,
   b) a rapid reduction in CRP, as measured using a standard CRP assay.
Item 17. The method according to any of the above items, wherein said patient achieves a sustained response after the end of treatment (for example 3 months after the end of the treatment), as measured by inflammatory lesion count, Hidradenitis Suppurativa Clinical Response (HiSCR), Numerical Rating Scale (NRS), modified Sartorius HS score, Hidradenitis Suppurativa - Physician Global Assessment (HS-PGA), or Dermatology Life Quality Index (DLQI).
Item 18. The method according to item 17, wherein said patient achieves a sustained response after the end of treatment (for example 3 months after the end of the treatment), as measured by the simplified HiSCR (sHiSCR).
Item 19. The method according to any one of the above items wherein the LTA4H inhibitor is administered at a dose of about 10 mg to about 30mg twice a day.
Item 20. A LTA4H inhibitor for use in the treatment and/or the prevention of hidradenitis suppurativa (HS) in a patient in need of such treatment and/or prevention.
Item 21. A LTA4H inhibitor for use according to claim 20, wherein said LTA4H inhibitor is a compound of Formula (I), or a pharmaceutically acceptable salt thereof: 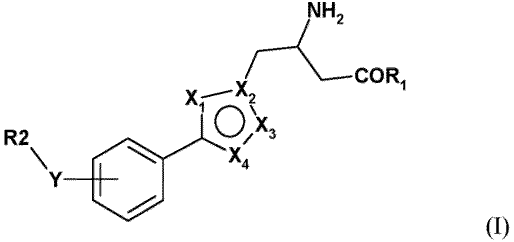 wherein,
   R1 is OH or NH₂;
   Y is O, S or CH₂;
   X1, X2, X3 and X4 are N; or
   X1, X2, X3 and X4 are selected from N, NH, C, CH and O with the proviso that at least two of
   X1, X2, X3 or X4 are N or NH;
   R2 is C₁-C₆ alkyl optionally substituted by phenyl; C₃-C₆ cycloalkyl; phenyl optionally being substituted by halogen, cyano, C₁-C₆ alkyl optionally substituted by halogen, C₁-C₆ alkoxy, or a 5-6 membered heteroaryl ring containing 1 to 3 heteroatoms selected from N, O and S; or a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen.
Item 22. A LTA4H inhibitor, for use according to item 20 or 21, wherein said LTA4H inhibitor is (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof.
Item 23. A LTA4H inhibitor for use in accordance to any one of items 20 to 22, wherein the LTA4H inhibitor is disposed in a pharmaceutical formulation, wherein said pharmaceutical formulation comprises one or more pharmaceutically acceptable carriers, each of which is independently selected from a filler, a lubricant, a binder, a desintegrant and a glidant.
Item 24. A LTA4H inhibitor for use in accordance to item 23, wherein the pharmaceutical formulation is in tablet or capsule form.
Item 25. A pharmaceutical composition comprising a LTA4H inhibitor which is a compound of Formula I, or a pharmaceutically acceptable salt thereof, together with one or more pharmaceutically acceptable carriers for use in the treatment and/or prevention of HS in a patient in need of such treatment and/or prevention.
Item 26. A LTA4H inhibitor for use according to any one of items 20 to 24 or a pharmaceutical composition for use in accordance to item 25, wherein the LTA4H inhibitor is administered at a daily dose of about 10 mg to about 100mg.
Item 27. A LTA4H inhibitor for use according to any one of items 20 to 24 and 26, or a pharmaceutical composition for use in accordance to item 25 or 26, wherein the LTA4H inhibitor or a pharmaceutical composition comprising it, is administered in combination with one or more second therapeutic agents.
Item 28. A combination product comprising a therapeutically effective amount of a compound of Formula (I) or a pharmaceutically acceptable salt thereof and one or more therapeutic agents, for use in the treatment and/or prevention of HS in a patient in need of such treatment and/or prevention.
Item 29. A LTA4H inhibitor for use according to any one of items 20 to 24 and 26, a pharmaceutical composition for use according to any one of items 25 and 26, wherein the patient is additionally treated with at least one topical medication and at least one antiseptic in combination with the LTA4H inhibitor.
Item 30. A LTA4H inhibitor for use according to any one of items 20 to 24, 26, 27 and 29, a pharmaceutical composition for use according to any one of items 25 to 27 and 29, or a combination according to item 28 or 29, wherein, prior to treatment with the LTA4H inhibitor, the patient has not been previously treated with a systemic agent or a topical treatment for HS.
Item 31. A LTA4H inhibitor for use according to any one of items 20 to 24, 26, 27 and 29, a pharmaceutical composition for use according to any one of items 25 to 27 and 29, or a combination according to item 28 or 29, wherein the patient is selected according to one of the following criteria:
   a) the patient has moderate to severe HS;
   b) prior to treatment with the LTA4H inhibitor, the patient has an HS-PGA score of ≥3;
   c) prior to treatment with the LTA4H inhibitor, the patient has at least 3 inflammatory lesions; or
   d) prior to treatment with the LTA4H inhibitor, the patient does not have extensive scarring (<10 fistulas) as a result of HS.
Item 32. A LTA4H inhibitor for use according to any one of items 20 to 24, 26, 27 and 29-31, a pharmaceutical composition for use according to any one of items 25 to 27, and 29-31, or a combination according to any one of items 27 to 31, wherein said patient achieves by week 16 of treatment at least one of the following:
   a) a simplified HiSCR;
   b) a reduction in HS flares;
   c) a NRS30;
   d) a reduction of ≤ 6 as measured by the DLQI; and/or
   e) an improvement in DLQI.
Item 33. A LTA4H inhibitor for use according to any one of items 20 to 24, 26, 27, and 29-31, a pharmaceutical composition for use according to any one of items 25 to 27, and 29-31, or a combination according to any one of items 27 to 31, wherein by week 16 of treatment, at least 40% of said patients achieve a simplified HiSCR; or at least 25% of said patients achieve an NRS30 response; or less than 15% of said patients experience an HS flare.
Item 34. A LTA4H inhibitor for use according to any one of items 20 to 24, 26, 27, and 29-31, a pharmaceutical composition for use according to any one of items 25 to 27, and 29-31, or a combination according to any one of items 27 to 31, wherein the patient has at least one of the following as early as one week after the first dose of the LTA4H inhibitor:
   a) a rapid reduction in pain, as measured by VAS or NRS, and
   b) a rapid reduction in CRP, as measured using a standard CRP assay.
Item 35. A LTA4H inhibitor for use according to any one of items 20 to 24, 26, 27 and 29-34, a pharmaceutical composition for use according to any one of items 25 to 27 and 29-34, or a combination according to any one of items 27 to 34, wherein said patient achieves a sustained response after the end of the treatment (for example 3 months after the end of the treatment), as measured by inflammatory lesion count, Hidradenitis Suppurativa Clinical Response (HiSCR), Numerical Rating Scale (NRS), modified Sartorius HS score, Hidradenitis Suppurativa - Physician Global Assessment (HS-PGA), or Dermatology Life Quality Index (DLQI).
Item 36. A LTA4H inhibitor, a pharmaceutical composition, or a combination according to item 35, wherein said patient achieves a sustained response after the end of treatment, for example 3 months after the end of the treatment, as measured by the simplified HiSCR (sHiSCR).
Item 37. A LTA4H inhibitor for use according to any one of items 20 to 24, 26, 27, and 29-34, a pharmaceutical composition for use according to any one of items 25 to 27, and 29-34, or a combination according to any one of claims 27 to 34, wherein the LTA4H inhibitor is administered at a dose of about 10 mg to about 30mg twice a day.

### General

The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural references unless the context clearly dictates otherwise. Unless defined otherwise, all technical and scientific terms used herein have the same meaning as commonly understood by one of ordinary skill in the art to which this disclosure belongs. All patents and publications cited in this specification are incorporated by reference. The following Examples are presented in order to more fully illustrate the preferred embodiments of the disclosure. These examples should in no way be construed as limiting the scope of the disclosed subject matter, as defined by the appended claims.

### EXAMPLES

### Abbreviation

- AE: adverse effect
- AUC: area under the curve
- AUClast: area under the plasma (or serum or blood) concentration-time curve from time zero to time of last quantifiable concentration (mass x time/volume)
- AUCtau: area under the plasma (or serum or blood) concentration-time curve from time zero to the end of the dosing interval tau (mass x time/volume)
- b.i.d. or BID: twice daily
- CI: confidence interval
- Cmax: maximum concentration after drug administration
- Cmax, ss: maximum concentration at steady state
- ECG: electrocardiogram
- EoS: end of study
- PK: Pharmacokinetics
- PD: Pharmacodynamics
- q.d. or QD: once a day
- ,ss: at steady state
- Tmax: time limit to reach maximum concentration after drug administration
- T1/2: the terminal elimination half life

### Example 1: (S)-3-amino-4-(5-(4-((5-chloro-3-fluoropyridin-2-yl)oxy)phenyl)-2H-tetrazol-2-yl)butanoic acid (Crystalline Form B)

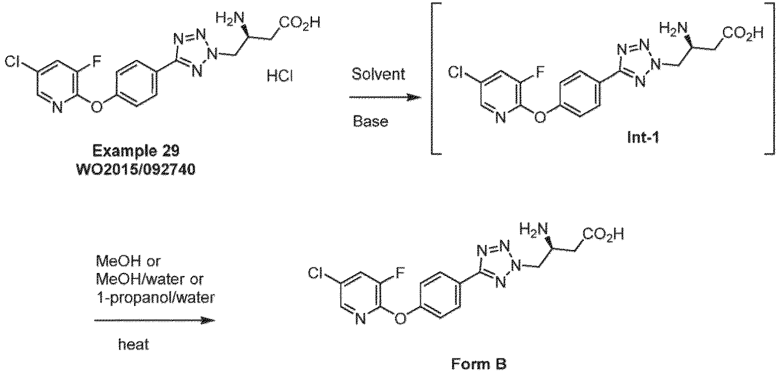

Example 29 as described in WO2015/092740 (28 g, 35 mmol) and a solvent mixture containing 360 g water and 40 g THF was mixed together and stirred for 20 minutes. The mixture was filtered and the filtrate was adjusted to pH = 5 with aqueous NaHCO₃. The stirring was continued for 18 h before the mixture was filtered to afford the free acid (Int-1) in wet cake 25.6 g, which was used for preparation of polymorph Form B without further purification. 505 mg of the free acid (Int-1) are weighed into a 20 ml glass vial and 6 mL of methanol are added. The slurry is heated to 50°C and stirred for 4 days using a magnetic stirrer. The suspension is cooled to room temperature and filtered. The recovered solid is dried at 40°C for 2.5 h under vacuum. The white solid was analyzed by XRPD, DSC and TGA (Figures 1-3 respectively).

Alternative methods for making crystalline Form B are described in Patent application No. PCT/CN2018/000278, filed on July 31st 2018 (Attorney docket number PAT058189-WO-PCT).

### Powder X-Ray Diffraction

X-ray powder diffraction (XRPD) patterns were obtained using a Bruker Discovery D8 in reflection geometry (Figure 1). Powders were analyzed using a zero background Si-wafer sample holder. The radiation was Cu Kα (l = 1.5418 Å). Patterns were measured between 2° and 40° 2theta.

**Table 1**

| **X-ray powder diffraction data for crystalline form B** | | |
|---|---|---|
| Angle (°2theta) | Intensity (d value Angstrom | rel. Intensity (%) |
| 10.9 | 2863 | 23 |
| 11.3 | 5874 | 47 |
| 12.8 | 5903 | 48 |
| 15.2 | 3080 | 25 |
| 17.1 | 1900 | 15 |
| 19.7 | 6203 | 50 |
| 20.0 | 2950 | 24 |
| 20.3 | 4949 | 40 |
| 20.5 | 2602 | 21 |
| 21.0 | 2526 | 20 |
| 22.6 | 9030 | 73 |
| 24.1 | 12400 | 100 |
| 24.4 | 3104 | 25 |
| 25.1 | 7318 | 59 |
| 26.3 | 8074 | 65 |
| 28.5 | 5291 | 43 |
| 29.3 | 1891 | 15 |
| 30.0 | 2649 | 21 |
| 36.4 | 2085 | 17 |
| 39.1 | 1308 | 11 |

### DSC:

Differential scanning calorimetry was conducted for each crystalline form using a TA Instruments (DSC 2500) (Figure 2). For each analysis, 2-4 mg of sample was placed in an aluminium T-zero crucible that closed with a pin-hole lid. The heating rate was 10°C per minute in the temperature range between 30 and 300°C. Temperatures are reported in degrees Celsius (°C) and enthalpies are reported in Joules per gram (J/g). Plots are showing endothermic peaks as down. The endothermic melt peak (melting point) was evaluated for extrapolated onset temperature. The accuracy of the measured sample temperature with this method is within about ±1 °C, and the heat of fusion can be measured within a relative error of about ±5%.

Illustrative DSC trace generated using crystalline Forms B is shown in Figure 2.

### Form B: Melting endotherm: Tₒₙₛₑₜ = 197.4° C (melting under decomposition)

### Thermogravimetric Analysis (TGA):

TGA curves were obtained using a TA-instrument Q5000. 5-15mg of sample was placed into an aluminum crucible and sealed hermetically. The sealed crucible was pierced by the robotic auto sampler immediately before analysis. The TGA curve was measured at 10°C/min between 30-300°C. The LoD (Loss of drying) was calculated between 40°C and 150°C. The weight loss is plotted against the measured sample temperature. Temperatures are reported in degrees Celsius (°C) and weight loss in %.

Illustrative TGA trace generated using crystalline Forms B is shown in Figure 3.

### Example 2:

| **Formulation of compound of Example 1 as a capsule** | | | | |
|---|---|---|---|---|
| **Ingredient** | **Amount per capsule (mg)** | | | **Function** |
| | **1 mg** | **5 mg** | **50 mg** | |
| **Capsule fill** | | | | |
| Compound of Example 1 | 1.00 | 5.00 | 50.00 | Active Ingredient |
| Mannitol | 98.75 | 104.75 | 145.95 | Filler |
| Cellulose, microcrystalline / Microcrystalline Cellulose | 40.00 | 30.00 | 50.00 | Filler |
| Crospovidone | 5.00 | 5.00 | 12.50 | Disintegrant |
| Hypromellose | 3.00 | 3.00 | 7.50 | Binder |
| Magnesium Stearate¹ | 1.50 | 1.50 | 2.70 | Lubricant |
| Silica, colloidal anhydrous / Colloidal silicon dioxide | 0.75 | 0.75 | 1.35 | Glidant |
| Water, purified (bulk) / Purified water² | --- | --- | --- | Suspending agent/ Solvent |
| **Capsule fill weight** | **150.00** | **150.00** | **270.00** | - |
| Capsule shell (theoretical weight)³ | 48.00 | 48.00 | 76.00 | - |
| **Total capsule weight** | **198.00** | **198.00** | **346.00** | - |

| | | | | |
|---|---|---|---|---|
| ¹ Vegetable origin ² Removed during the processing ³ The components of the capsule shell are given in Table below | | | | |

### Components of one capsule shell

| | |
|---|---|
| | **Capsule shell components** |
| | Gelatin |
| | Titanium dioxide (E171) |
| | Iron oxide (E172), red |
| E: refers to Official number used in the European Union for colorants | |

Regulation (EU) 231/2012: commission regulation (EU) laying down specification for food additives. Capsules are purchased from Lonza (now Capsugel)

### Example 3: Analysis of HS patient skin biopsies indicates strong activation of the 5-Lipoxygenase pathway in leukocytes of lesions

Neutrophils and Macrophages, the main cellular target for LTA4H inhibitors, are abundant in HS lesions. Histological analysis of HS lesions by H&E staining revealed abundance of myeloid cells such as neutrophils and macrophages in lesions of HS patients. Three-µm-thick paraffin sections were cut and stained with hematoxylin and eosin. Automated immunohistochemical stainings for 5-lipoxygenase (clone: EP6072(2), Abcam, UK) and CD68 (clone KP-1, Dako, Denmark) were performed on Ventana Discovery XT immunostainer (Roche Diagnostics, Switzerland). Specific isotype controls were used as negative controls. All biopsy samples were digitalized using ScanScope XT slide scanner (Aperio, Leica Biosystems, Switzerland) with objective x40.

Macrophages were identified by anti-CD68 staining. Co-Staining with an antibody against the 5-lipoxygenase demonstrated strong nuclear expression of the 5-Lipoxygenase enzyme in myyeloid cells found in the lesional skin of HS patients. High magnification images revealed that expression of the 5-Lipoxygenase enzyme at the nuclear membrane in many macrophages, a sign of pathway activation (Christmas et al., 1999 "Differential localization of 5- and 15-lipoxygenases to the nuclear envelope in RAW macrophages." J. Biol. Chem. p. 25594-8.). 5-Lipoxygenase translocation to the nuclear membrane activates the enzyme and provides its biosynthesis product LTA4 which is the substrate of LTA4H that converts it into LTB4. (Figures 4, 5, 6)

**Figure 4****:** Lesional biopsy obtained from HS patient. Paraffin section stained with hematoxylin and eosin revealed severe mixed inflammatory infiltrates containing high numbers of polymorphonuclear cells and macrophages (scale bar indicates 4 mm).

**Figure 5****:** HS lesional skin biospies were stained immunohistochemically for 5-Lipoxygenase. The staining revealed strong expression of 5-Lipoxygenase in myeloid cells. The 5-Lipoxygenase expression pattern was either diffuse nuclear (short arrows) or at the nuclear membrane (long arrows, scale bar indicates 50 µm).

**Figure 6****:** A representative photomicrograph illustrating 5-Lipoxygenase (brown) localized at the nuclear membrane in multinucleated giant cell CD68+ (red, scale bar indicates 30 µm).

### Example 4: Lipidomics analysis of HS patient skin biopsies

Frozen human skin biopsies from healthy skin or discard HS lesions (on average 500 mg) were pulverized using mechanical force (Covaris) and homogenized in 4 mL of methanol containing deuterium labelled internal standards (ISTDs) using a bead-beating device (2×20sec cycles of 5000 rpm, Precellys) maintained at low temperature. Then, samples were kept at -20°C/45 min to allow for protein precipitation and centrifuged at 13000 rpm for 10min at 4°C. The supernatants were then evaporated (Genevac) until complete dryness and reconstituted in 70 µL of methanol: water (50%/50%; v/v) followed by injection in the LC-MS/MS system. Calibration curves were freshly prepared by serial dilution of pure standards in methanol: water (50%/50%, v/v) containing ISTDs and quantitation was done by comparing metabolite to ISTD peak area ratios in samples to that of calibrators.

Two analytical platforms were employed for data acquision depending on instrument availability: 1) Waters H class UPLC system coupled to a Waters XevoTQS triple quadrupole mass (QQQ) spectrometer and 2) a Dionex Ultimate 3000RS UPLC coupled to a Sciex6500+ QQQ. Separation of lipids was achieved using reverse-phase columns ACQUITY UPLC BEH C18 1.7µm, 2.1 × 50 mm and CORTECS UPLC C18 1.6µm, 2.1 × 50 mm heated at 40°C, respectively. The flow rate was set to 0.180 mL/min and the mobile phase consisted of water-acetic acid (99.99/0.01; v/v) (A) and methanol-acetonitrile-acetic acid (B) (50/50/0.01; v/v/v). The eluent gradient was maintained at 45% of B for 2 min, follow by 45%-65% of B during 2-16 min, from 65%-100% during 16-19 min and 100% ofB from 19-22 min. From 23-30 min the column was re-equilibrated with initial conditions. Ionization was performed using an ESI source operated in negative ion mode. Fragmentation was tuned for each compound individually.

For peak picking and quantitative analysis vendor specific software was used (TargetLyx for Waters and Analyst/Multiquant for Sciex data). Further data processing including missing values imputation (replacement by the group average), logarithmic transformation (base 2), correction of batch effects (based on scaling by mean of each sample) and group comparisons (empirical Bayes moderated t-Tests) were performed in R (version 3.4.2) with packages Metabolomics and NormalizeMets (available from CRAN). (Anal. Chem., 2015, 87 (7), pp 3606-3615). (Figures 7 and 8)

Lipids were extracted from skin biopsies of 14 healthy and 16 HS patients and analyzed by LC-MS/MS.

**Figure 7****:** The product of LTA4H, LTB4 was significantly upregulated in lesional skin as compared to non-lesional skin (depicted in log₂ of mean scaled concentrations [fmol/mg]).

**Figure 8****:** 5-Lipoxygenase pathway mediator 5S,6R DiHETE was also elevated indicating strong activation of the 5-Lipoxygenase pathway in HS lesions (depicted in log₂ of mean scaled concentrations [fmol/mg]). *** p<0.001 using a two-tailed unpaired t-test.

### Example 5: Transcriptomics analysis of HS skin biopsies versus healthy skin biopsies

Transcriptomics analysis of 18 HS skin biopsies versus 8 healthy skin biopsies shows transcriptomic upregulation of 5-Lipoxygenase pathway genes.

From snap frozen skin tissue, a homogenate was prepared using recommended buffers from Qiagen Rneasy mini kit. The total RNA of the cells was extracted according to manufacturers protocol. cDNA of the samples was prepared from the same starting amount of RNA using a High capacity cDNA reverse Transcription Kit (Applied Biosystems). Samples were processed by CiToxLAB France on Affymetrix HG_U133_Plus2 microarrays. RMA normalized data was analyzed using GeneSpring 11.5.1 (Agilent Technologies, Santa Clara, CA) and the results were interpreted using Illumina BaseSpace Correlation Engine software and Qiagen IPA. Initially, the data were subject to standard QC control by CiToxLAB and in GeneSpring (PCA, hybridization controls). Subsequently, it was filtered on expression levels to probesets above the 20th percentile in 100% of the samples in any one of the conditions before further analysis. Lesional skin of HS patients showed elevated levels of 5-Lipoxygenase pathway genes including LTA4H. LTA4H was highly expressed in skin of both healthy and lesional skin (Figure 9).

**Figure 9** shows normalized expression of target genes A significant upregulation of the 5-Lipoxygenase genes ALOX5, ALOX5AP and LTA4H of 18 HS patient samples versus 8 healthy control samples could be determined, suggesting an upregulation of LTB4 biosynthesis in agreement with lipidomics data. *** P<0.001; ** P<0.01; * P<0.05 applying a two-tailed unpaired t-test.

### Example 6: Compound of Example 1 and compound of embodiment 3D suppress the biosynthesis of pro-inflammatory LTB4 in stimulated skin biopsies of HS patients

Skin biopsies from HS patients were cultivated in cell culture medium for 2h in the presence or absence of 5uM of LTA4H inhibitors selected from the compound of example 1 and the compound of embodiment 3D. Biopsies were then stimulated for 2h with 10ug/mL ionophore to induce full activation of 5-lipoxygenase pathway in inflammatory cells of lesions. Skin biopsies were then harvested, lipids extracted and analyzed for generation of LTB4 by LC-MS/MS as described above. Stimulated skin biopsies of HS lesions generated a large amount of the lipid mediator LTB4. Skin biopsies that were treated with compound of example 1 or compound of embodiment 3D released almost no LTB4. **(****Figures 10 and 11****)**

Lesional skin biopsies of HS patients were left untreated or fully stimulated with ionophore to induce LTB4 biosynthesis in the presence or absence of a LTA4H inhibitor

**Figure 10****:** compound 1 is compound of example 1;

**Figure 11****:** compound 2 is compound of embodiment 3D

Presence of a LTA4H inhibitor fully suppressed the LTB4 producing capability of lesional skin biopsies of HS patients. LTB4 quantities are given in fmol/mg tissue. Shown are representative results obtained with one donor per compound out of several tested.

**Figure 12****:** Mice were treated with either vehicle, 3 mg/kg or 10 mg/kg compound 2 (compound of embodiment 3D). 22 hours after the last dose, blood was obtained, diluted 1:3 and stimulated for 15 min ex-vivo with 10 mg/ml calcium ionophore to induce LTB4 production. Release of LTB4 was quantified via LTB4 EIA and demonstrated over 90% inhibition of LTB4 in both treated groups. Depicted are averages of 4 mice per group +/- standard error of the mean.

### Example 7: Clinical data from first in Human (FIH) trial

Compound of example 1 has been studied in a FIH study designed to characterize its preliminary safety, tolerability, and PK in adult healthy subjects. The study consisted of a part 1, single ascending dose (SAD) and a part 2 multiple ascending dose (MAD). Compound of example 1 was administered orally in single either QD or BID, in fed or fasted conditions over a dose range of 5 mg to 2 times 100 mg. Compound of example 1 was also administered orally in multiple-dose administration, in fasted conditions for 12 days over a dose range of 5 mg QD to 80 mg BID.

### Part 1 (SAD)

Part 1 was a randomized, subject-blinded and Investigator-partial-blinded, placebo-controlled, single ascending oral dose study. The dose was administered either in one take or split into two intakes at 12 hours apart (N =69). Eight subjects were randomized into each cohort (except in Cohort 7 where seven subjects were randomized and in Cohort 9 where six subjects were randomized) to receive either compound of example 1 or matching placebo in a 6:2 ratio (active: placebo), testing nine dose levels.

Subjects were assigned to one of the following cohort:
Cohort 1: Single oral dose of 5 mg or matching placebo
Cohort 2: Single oral dose of 10 mg or matching placebo
Cohort 3: Single oral dose of 20 mg or matching placebo
Cohort 4: Single oral dose of 30 mg or matching placebo
Cohort 5: Single oral dose of 45 mg or matching placebo
Cohort 6: Single oral dose of 70 mg or matching placebo
Cohort 7: total oral dose of 140 mg or matching placebo split into two doses of 70 mg taken 12h apart
Cohort 8: total oral dose of 200 mg or matching placebo split into two doses of 100 mg taken 12h apart
Cohort 9: total oral dose of 80mg or matching placebo split into two doses of 40 mg taken 12h apart

### Part 1 (Single ascending dose) design (qd) - Overview of the study

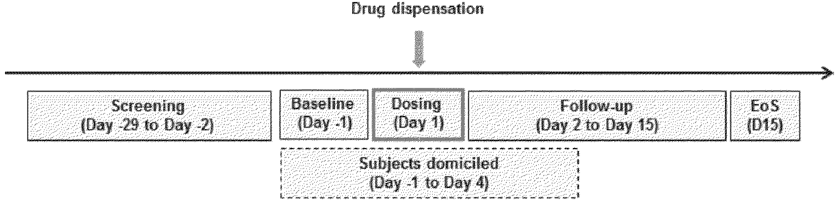

### Part 1 (Single ascending dose) design (Split daily intake) - Overview of the study

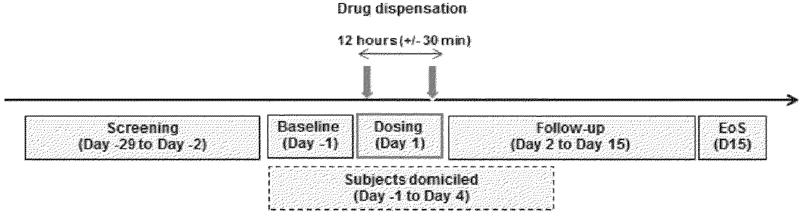

### Part 2 (MAD)

Part 2 was a randomized, subject-blinded and investigator-partial-blinded, placebo-controlled, MAD study in which, eight subjects each were randomized into five cohorts to receive either compound of example 1 or matching placebo in a 6:2 ratio (active: placebo).

Subjects were assigned to one of the following cohort:
Cohort 1: multiple once daily (qd) oral dose of 5 mg or matching placebo
Cohort 2: multiple once daily (qd) oral dose of 15 mg or matching placebo
Cohort 3: multiple oral daily dose of 20mg or matching placebo administered twice daily (bid)
Cohort 4: multiple oral daily dose of 40mg or matching placebo administered twice daily (bid)
Cohort 5: multiple oral daily dose of 80mg or matching placebo administered twice daily (bid)

### Part 2 multiple ascending dose design (qd) - Overview of the study

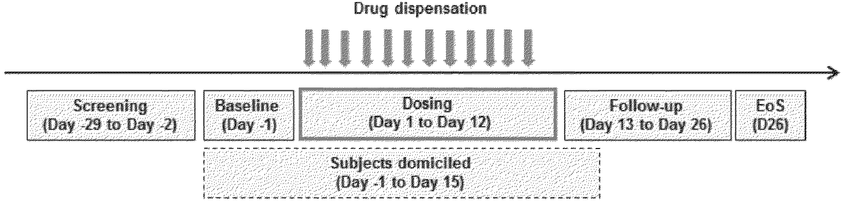

### Part 2 multiple ascending dose design (bid) - Overview of the study

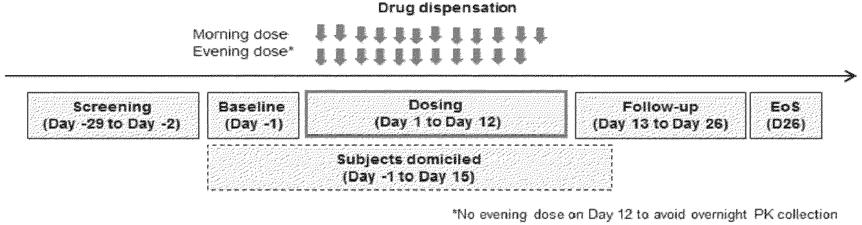

### Human safety and tolerability

In Part 1 (Single Ascending Dose, SAD) of the above study, healthy subjects received a single dose of compound of example 1 up to a maximal total daily dose of 200 mg (two times 100 mg administered 12 h apart). In Part 2 (Multiple Ascending dose, MAD) of study, the subjects received doses up to 80 mg BID for 12 days. No adverse events led to study discontinuation. The highest doses in both parts were safe and no maximal tolerated dose was established.

Some subjects who were treated with compound of example 1 or placebo in the SAD and MAD portions of this study experienced asymptomatic lipase elevations. Three treated subjects (one from the SAD part at the lowest dose of 5 mg, two from the MAD at the highest dose of 80 mg BID) and one placebo treated subject experienced increases in lipase. In the three subjects with lipase elevations, the lipase increase was accompanied by mild amylase increase (≤1.5 ULN). All subjects were asymptomatic and events were transient, rapidly returning to normal levels while subjects continued to receive compound of example 1. No significant findings in physical exam, vital signs or ECGs have been related to compound of example 1.

Therefore, compound of example 1 was well tolerated in healthy subjects at doses up to 80 mg bid (160 mg daily dose) over 12 days.

### Human pharmacokinetic data

Pharmacokinetic behavior of compound of example 1 was evaluated in healthy subjects following single and multiple oral doses. The PK parameters calculated were standard parameters used for measuring drug exposure in the systemic circulation after receiving single or multiple doses of compound of example 1.

### Part 1 (SAD)

The mean plasma concentration time profiles for compound of example 1 are shown in Figure 13.

Following single oral administration of the compound at 5, 10, 20, 30, 45, 70, two times 70 (split intake, 2 identical doses were administered 12 h apart), two times 100 (split intake) and two times 40 mg (split intake), the plasma exposure to compound of example 1 increased with dose and a median Tmax ranging from 1 to 1.5 hours post dose indicated a fast absorption. After the concentration peak plasma concentrations decreased initially very rapidly; at later time points the rate of concentration decline decreased strongly and the mean apparent elimination half-life (T1/2) ranged from 245 to 513 hours. With an increase in dose from 5 to 100 mg (i.e. 20-fold), Cmax increased by 21.7-fold; AUC0-24h increased by 75 fold, with a 40-fold increase in daily dose (5 mg to 100 mg bid). The percentage coefficient of variation (CV%) ranged from 18.5 to 41.4 for Cmax and 6.4 to 26.3 for AUC0-24h.

For the dose range of 5 mg to 2 times 100 mg (40-fold) for AUC and 5 mg to 100 mg for Cmax (20-fold), the estimated slope and the corresponding 90% CI for Cmax was 1.08 (1.01, 1.15), for AUClast the slope was 1.00 with 90% CI (0.957; 1.04) and for AUC0-24 the slope was 1.20 with 90% CI of (1.15, 1.24). Dose proportionality over the whole dose range was demonstrated for AUClast but not for Cmax and AUC0-24h where data suggest a slightly over proportional increase of exposure with dose. However, for Cmax and AUC0-24h PK can be considered dose proportional for an up to 4.50-fold and 2.57-fold increase in dose, respectively.

### Part 2 (MAD)

The mean plasma concentration time profiles of the compound are shown in Figure 14a (Day 1) and Figure 14b (Day 12).

Following oral administration of the first dose of compound of example 1 on Day 1, for all dose groups (5 and 15 mg qd fasted; 20, 40 and 80 mg bid fed), the plasma concentrations of compound of example 1 increased in a dose dependent manner with a median Tmax ranging from 1 to 2.5 hours.

With an increase in the dose of compound of example 1 from 5 mg qd to 80 mg bid (i.e. 16-fold per dosing interval), Cmax and AUC on Day 12 (AUC0-12h,ss for 20, 40 and 80 mg bid cohorts and AUC0-24h,ss for 5 and 15 mg qd cohort) increased by 17.9-fold and 20.9-fold respectively. For the dose range of 5 to 80 mg (16 fold), the estimated slope and the corresponding 90% CI for Cmax,ss was 1.19 (1.08,1.30) and for AUCtau,ss the slope was 1.07 with 90% CI (1.01; 1.12). Dose proportionality over the whole dose range was not demonstrated for both Cmax,ss and AUCtau,ss. However, Cmax,ss and AUCtau PK can be considered dose proportional for and up to 2.12 and 6.29 fold increase in dose, respectively. Dose proportionality criteria were met for AUCtau over the dose range 5 mg qd to 40 mg bid. The % CV ranged from 17.1 to 30.2 % for Cmax and 11.7 to 18.1 for AUC0-24h.

Across the investigated dose range Cmax,ss was between 1.39 to 1.19 times greater than after single dose (mean Cmax (Day 12)/ Cmax (Day 1)). For AUC (AUC0-12 and AUC0-24), the average ratios were between 2.08 to 1.27. This indicates minor accumulation of compound of example 1 from day 1 to steady state. For the lowest dose of 5 mg qd 2.1-fold mean accumulation to steady state was observed but for all higher doses accumulation was ≤ 1.4-fold. Little accumulation on day 1 of the bid regimen (mean Racc, day 1 from 1.09 - 1.29) suggests that time to steady state is short. In 4 of the 5 dose groups morning pre-dose concentrations are higher on Day 3 as compared to Day 2, but the concentration differences are small. From Day 3 to Day 4 no consistent or major increase in morning pre-dose concentrations is observed. This demonstrates that steady state is achieved fast i.e. approximately on day 3.

For BID oral dosing of compound of example 1, in the fed state, steady state exposure is reached within 3-4 days of dosing. At steady state, dose proportionality criteria were met for AUCtau from 5 mg QD to 40 mg BID.

### Human pharmacodynamic data

### PD blood biomarker: Leukotriene B4 (LTB4) in blood

### Determination of Leukotriene B4 (LTB4) in human plasma of ex vivo stimulated whole blood

In animal models the degree of LTB4 inhibition in ex-vivo stimulated blood correlated well with degree of therapeutic effect (Figure 10), in particular reduction of neutrophils in inflamed tissues. The peripheral PD readout of ex-vivo stimulated blood LTB4 is predictive for therapeutic efficacy and hence suitable to monitor target inhibition in the FIH trial.

Figure 10: Mice were treated with either vehicle, 3 mg/kg or 10 mg/kg compound 2. 22 hours after the last dose, blood was obtained, diluted 1:3 and stimulated for 15 min ex-vivo with 10 mg/ml calcium ionophore to induce LTB4 production. Release of LTB4 was quantified via LTB4 EIA and demonstrated over 90% inhibition of LTB4 in both treated groups. Depicted are averages of 4 mice per group +/- standard error of the mean.

Therefore, LTB4 concentrations were determined in plasma of ex-vivo stimulated whole blood by a LC-MS/MS method.

### Bioanalytical method for LTB4

Ex-vivo stimulation was performed at the clinical site according to the following procedure: Whole blood (500µL) were stimulated for 30 min using Calcium Ionophore A23187. Then plasma supernatant is harvested, and frozen at -80°C for at least 24 hours prior to use.

LTB4 quantification by LC-MS/MS was performed as follow:
Human plasma was processed with organic precipitation followed by separation by reverse phase high performance liquid chromatography with tandem mass spectrometric detection.

### Chromatography:

Reversed phase separation on Agilent Technologies 1290 Infinity HPLC and Zorbax SB-C18, Rapid Resolution HD 1.8µm (100 × 2.1mm) column at 60°C and flow rate of 400 µL/min Total analytical run time of 11.0 minutes.

### Detection:

AB Sciex QTrap 5500 MS/MS Turbo Spray Ion Drive negative ion mode.

Sample from each subject and time point was prepared and analyzed in three aliquots. Aliquot one and two contained plasma from *ex-vivo* stimulated whole blood, while aliquot three as negative control contained plasma from non-stimulated whole blood. Samples with CV>25% for the first two aliquots and/or with detected values in aliquot three were excluded from the results.

LTB4 concentration data were calculated as mean of concentration of aliquot one and two. Concentration of aliquot three was not be included in the calculation. The change from baseline and percent inhibition (percent of baseline) for each biomarker was calculated. Baseline was the mean of the Baseline and Day 1, -1h measurements.

To avoid being treated as missing values when calculating changes from baseline and percent inhibition, values below the LLOQ were replaced by 0.5 × LLOQ and values above the upper limit of quantification (ULOQ) were replaced by 1.5 × ULOQ.

### Results:

Results from the first cohorts of the SAD indicated that blood cell stimulated LTB4 release remained at 25-50% reduction from baseline at 24 hours with 5 mg. Inhibition at 24 hours was observed to increase with increasing dose. An approximately 50% inhibition at 24 h after dosing was observed with the 10 mg dose and transient maximum inhibition (>90%) following Cmax was observed at the 20 mg dose. (Figure 15)

Additional cohorts in the SAD part confirmed the dose dependent inhibition of LTB4. Approximately 81%, 86% and 89% inhibition at 24h after dosing was observed with the 30 mg, 45 mg and 70 mg dose, respectively.

Steady state data from cohorts 1 to 3 in the MAD part, confirm the dose dependent inhibition of LTB4. Approximately 75%, 90% and 99% inhibition was observed in samples collected pre-dose at Day 9 (steady state) with the 5 mg (Q.D.), 15 mg (Q.D.) and 20 mg (B.I.D.) dose, respectively. (Figure 16)

Target inhibition of 90-99% was maintained at 15 mg (Q.D.) and 20 mg (B.I.D.) doses, in the MAD part of the study.

In conclusion, steady state leukotriene A4 hydrolase target inhibition assessed ex-vivo in blood samples was on average 75% at 5mg QD, 90% at 15mg QD and 99% at 20mg QD bid.

Blister fluid biomarker: Leukotriene B4 (LTB4) in blood in cantharidin induced blister-fluid.

### Cantharidin induced skin blisters

To assess the PD effect of compound of example 1 on a potential target organ, skin blisters were induced on the arm of subjects in one selected cohort of SAD part and selected cohorts of MAD part.

In parts 1 and 2 of the clinical study (SAD and MAD), skin blisters were induced on the arm of subjects in selected cohorts. For this 10 mm diameter filter paper discs humected with a solution of cantharidin (such as cantharone) were applied on the arm of the subject, inducing an epidermal blister, and the blister fluid was collected approximately 24 hours (+/- 1hr) after the application.

Blister fluid samples were collected at Day 1 pre-dose and Day 2 pre-dose in SAD cohort 4 as well as Day 1 pre-dose and Day 9 predose in MAD cohort 2-5.

### Sample preparation for analysis of Leukotrienes and Lipoxin A4 in human blister fluid

Blister fluid samples were received frozen on dry ice and stored at -70°C or below until analysis. On the day of analysis, samples were taken out of the freezer and immediately extracted after Thawing.

### Procedure for Leukotrienes and Lipoxin A4 in human blister fluid

90 µL of blister fluid (sample or QC sample) was enriched with 5 µL of internal standard solution IS-L-2 (deuterated Leukotrienes and lipoxin at 50ng/mL) and vortexed in a micro centrifuge tube. 90 µL of methanol with 0.1% formic
acid was added. This was followed by 5 minutes vortex mixing and 10 minutes sonication on ice. The samples were incubated at -20°C in a freezer for 40 minutes. This was followed by centrifugation at 16,000rpm (27,500xg) at 4°C for 20 minutes. 160 µL of the supernatant was transferred onto Millipore 5kDa MWCO filters. MWCO filters have previously been cleaned by centrifuging 500 µL methanol/water 1/1 thru the filters. The loaded filters were centrifuged at 16000rpm (27500xg) at 4°C for 40 minutes (or until all liquid was filtered). 140 µL of filtrate was transferred to HPLC glass vials, combined with 108 µL of water and vortexed briefly. If volume of blister fluid was less than 90 µL, water was used to bring the volume up to 90 µL and the dilution factor was noted.

### High Performance Liquid Chromatography

HPLC was performed using an Agilent UHPLC system equipped with 2 pumps, one UHPLC pump (Model G4220B) for the analytical gradient and one quaternary pump (Model G1311B) as loading pump.

### Results

In Part 1 of the study, the blister fluid was assessed in the 30 mg treatment group with the sample collected at 24 hours and the mean concentration of compound of example 1 was found to be 4.67 ng/mL. The blister fluid concentrations were on an average approximately two-fold higher than the plasma concentrations at the same time and only 14% of the corresponding blood concentrations.

In Part 2 of the study, the blister fluid was analyzed in the 15 mg qd as well as the 20, 40 and 80 mg bid treatment groups with samples collected pre-dose on Day 9 (blister fluid) The mean concentrations of compound of example 1 increased with dose from 4.62 ng/mL at 15 mg qd to 233 ng/mL at 80 mg bid for blister fluid. Blister fluid concentrations were similar to plasma concentrations with blister fluid/plasma ratios around 2.

LTB4 inhibition in skin and plasma (% change from baseline) were recorded in Figure 17. 40 mg daily dose (20mg BID) shows nearly complete suppression in blood of LTB4 at steady state and the same dose achieved 90% suppression of LTB4 in human skin blister fluids, where the skin blister was induced by topical application of Cantharidin.

### Example 8: Efficacy and safety of (S)-3-amino-4-(5-(4-((5-chloro-3-fluoropyridin-2-yl)oxy)phenyl)-2H-tetrazol-2-yl)butanoic acid (Form B) in adult patients with moderate to severe HS.

Provided below are the details of the clinical trial design to demonstrate the efficacy of (*S*)-3-amino-4-(5-(4-((5-chloro-3-fluoropyridin-2-yl)oxy)phenyl)-2*H*-tetrazol-2-yl)butanoic acid compared to placebo.

### Clinical Study design

Blinding of subjects and investigators allows for an unbiased assessment of subjective readouts such as lesion counts in HS or global HS-PGA scores, as well as adverse events.

A randomized, subject and investigator blinded, placebo-controlled, multi-center and parallel-group study is run to assess efficacy, safety and tolerability of several active treatment compounds, such as compound of example 1, in subjects with moderate to severe hidradenitis suppurativa (HS). After a screening period of approximately 4-week, the treatment period is planned for 16 weeks and is followed by a safety follow-up of approximately 12 weeks. Subjects are given 20 mg BID p.o. of compound of example 1, or placebo BID p.o.

Subjects included in this study are adult male and female subjects of 18 to 65 years of age, presenting with moderate to severe HS diagnosed with recurrent inflammatory lesions for at least 12 months. The requirements for a subject to be included in the study is that he has at least 3 inflammatory lesions. Baseline evaluations may be started from Day -7 to allow completion of assessments on Day -1 prior to the treatment on Day 1. All baseline safety evaluation results must be available prior to dosing and meeting eligibility criteria. Randomization is done using a centralized Interactive Response Technology (IRT) system.

The selected primary clinical endpoint is simplified HiSCR (Hidradenitis Suppurativa Clinical Response) after 16 weeks of treatment.

On Day 113 (Week 17), after safety and other assessments have been performed, all subjects enter the follow-up period without administration of any drug. If medically justified, and if no potential safety concerns have been identified (after discussion with the sponsor), subjects may receive during this follow up period previously prohibited medication.

Safety and efficacy assessments are conducted at the follow-up visits on Day 141 (week 21) and Day 197 (week 29). Pharmacokinetics (PK) and pharmacodynamic (PD) are collected. The blinding is maintained for the investigator and the subject until the end of the study. The end of study visit occurs on Day 197 (Week 29), which includes study completion evaluations followed by discharge from the study.

Approximately 45 subjects are randomized, 30 on active and 15 on placebo. On Day 1, 20 mg of compound of example 1, or its corresponding placebo are provided to the subjects at the site. The subject is instructed by the site personal, how to take the drug (BID), including the need for fluid intake (preferably water) and to take the drug during or shortly after morning and evening meals. At baseline, drug administration and clinical assessments are performed as well as PK, pharmacodynamics (PD) and necessary safety assessments. subjects are discharged from the site on the same day after completion of all assessments provided and there are no safety concerns. Subjects return to the study center on Day 8 (Week 2), Day 15 (Week 3), and Day 29 (Week 5), and then in monthly intervals: Day 57 (Week 9), Day 85 (Week 13) and Day 113 (Week 17) respectively. Safety and selected efficacy assessments are conducted during these visits and PK/PD samples are collected.

The primary objective is to show preliminary efficacy of treatment with the compound of example 1, in HS subjects after 16 weeks of treatment in comparison to placebo. After the 16-weeks treatment period a follow up period for 12 weeks is included to observe a sustainability of the effect can be sustained or increased after 16 weeks of treatment.

A 2:1 randomization is planned based on the fact to favor exposure to active while limiting exposure of subjects to placebo.

The study includes multiple clinical endpoints, to better evaluate the properties of these selected endpoints:
- For this study, the simplified HiSCR was selected as the primary endpoint. The simplified HiSCR is defined as 50% decrease in the total number of abscesses plus inflammatory nodules, without any increase in draining fistulae.
- The inflammatory lesions of HS will be counted as individual lesions (inflammatory nodules, abscesses and draining fistulae) in the typical anatomical areas. In addition to the count, a global assessment scale (Hidradenitis suppurativa-physician global assessment or HS-PGA) as well as a composite score (Severity Assessment of Hidradenitis suppurativa score or SAHS) will be used.
- Several patient reported outcomes will be used, including the Dermatology Life Quality Index (DLQI). Finally, as from a subject's perspective skin related pain is the most important symptom, the numerical rating scale (NRS) for pain is included.

### Additional information on clinical assessments:

*HS-PGA* (Hidradenitis Suppurativa - Physician Global Assessment): The score will be used as exploratory objective to assess HS and was used and described in Kimball AB, Kerdel F, Adams D, et al (2012) Adalimumab for the treatment of moderate to severe Hidradenitis suppurativa: a parallel randomized trial. Ann Intern Med;157:846-55.

The *SAHS score* is a composite score (Hessam S, Scholl L, Sand M, et al (2018) A Novel Severity Assessment Scoring System for Hidradenitis Suppurativa. JAMA Dermatol;154(3):330-335.) and will be derived from the collected information for inflammatory lesion count, the fistulae count, and the NRS pain. In addition, the anatomical areas and the new or flared existing boils will be collected in both cohorts.

*Skin Pain* - *NRS* (numerical rating scale for pain): An NRS for skin related pain was used in adalimumab studies (Kimball et al. (2016) N Engl J Med 375:422-34) and will be used as skin or HS related pain is one of the highest burden for the patient (Matusiak et al (2017) J Am Acad Dermatol;76:670-5). The pain that is HS related will be recorded on average in the last 24 hours and at worst (in the last 24 hours).

Other *Patient reported outcomes* (PRO) will include Dermatology Life Quality Index (DLQI) as dermatology related Quality of life (QoL) tool with validated scores available in many countries and languages. It includes as well a Patient Global Assessment.

### Study objectives and endpoints

| ***Primary objective(s)*** | | | ***Endpoints related to primary objective(s)*** | | |
|---|---|---|---|---|---|
| • | To demonstrate efficacy of Example 1, compared to the placebo in moderate to severe inflammatory hidradenitis suppurativa (HS) patients | | • | Proportion of patients achieving clinical response evaluated by the simplified Hidradenitis Suppurativa Clinical Response (HiSCR) after 16 weeks of treatment | |

| ***Secondary objective(s)*** | | | ***Endpoints related to secondary objective(s)*** | | |
|---|---|---|---|---|---|
| • | To assess the safety and tolerability of compound of example 1 in patients with moderate to severe hidradenitis suppurativa (HS) | | • | Number and severity of AEs | |
| | | | • | Incidence of changes in safety laboratory, vital sign and ECG parameters | |
| | | | • | Physical examination and vital signs at baseline and repeatedly until study completion visit | |
| • | To evaluate the effect of compound of example 1 versus placebo on clinical outcome assessments over time | | • | Hidradenitis suppurativa - physician global assessment (HS-PGA) score over time | |
| | | | • | Inflammatory nodule count over time | |
| | | | • | Abscesses count over time | |
| | | | • | Draining fistulae count over time | |
| | | | • | Non-inflamed nodules count over time | |
| • | To evaluate the effect of the compound of example 1 to reduce HS flares versus respective placebo | | • | Proportion of patients who experience at least one flare over 16 weeks of treatment | |
| | | | | Flare is defined as an at least 25% increase in total abscess and inflammatory nodule counts (AN counts) with a minimum increase of 2 AN relative to baseline. | |
| | • | To assess the effect of the compound of example 1 compared to placebo on patient reported outcomes | | • | Dermatology Life Quality Index (DLQI) |
| | | | | • | Patient's Global Assessment |
| | | | | • | Proportion of patients achieving NRS30 after 16 weeks of treatment, among patients with baseline Skin Pain NRS ≥ 3 |
| | | | | • | Number of new boils or existing boils which flare up in the past four weeks. |

### Key inclusion criteria

- Male and female subjects, 18 to 65 years of age, with clinically diagnosed HS for at least 12 months prior to Screening
- A total of at least 3 inflammatory lesions, i.e., abscesses and/or inflammatory nodules, and
- No more than 10 fistulae, and
- At least two anatomical area need to be involved with HS lesions
- Subject must have a minimal body weight of 50Kg (inclusive)

### Key exclusion criteria

- Use of investigational drugs at the time of screening, or within 30 days of enrollment or 5 half-lives of enrollment or until the expected pharmacodynamic effect has returned to baseline, whichever is longer; or longer if required by local regulations.
- Use of IL12 and IL23 blocking biologics such as ustekinumab or guselkumab within the last 6 months prior to randomization/first treatment.
- Use of B-cell targeting or B-cell depleting biologics or similar such as rituximab or belilumab within 12 months; For patient who received these drugs earlier, B cellcount must be within normal range.
- Use of biological immunomodulating agents other than above (e.g., adalimumab, secukinumab, etanercept, infliximab, etc.) 3 months prior to randomization/ first treatment or 5 half-llives (whichever is longer);
- Use of rituximab or belilumab within 12 months;
- Use of any systemic treatment for HS in the last 4 weeks prior to randomization (such as retinoids or other immunmodulating therapies, e.g., methotrexate, cyclosporine A, corticosteroids).
- Use of cyclophosphamide within the last 6 months
- Use of systemic antibiotics for HS in the last week prior to randomization/first treatment
- If spironolactone or other antiandrogens (finasteride, cyproterone acetate, etc.) are used (for HS), only patients with a stable dose for the last 3 months and who are planning to continue for the duration of the study are eligible.
- Surgical treatment for HS in the last 4 weeks prior to randomization/first treatment. Surgical treatment does not include sporadic excisional biopsies
- Receipt of any high injected corticosteroid bolus (>1mg/kg) within 3 months

## Claims

1. A LTA4H inhibitor for use in the treatment and/or the prevention of hidradenitis suppurativa (HS) in a patient in need of such treatment and/or prevention.

2. A LTA4H inhibitor for use according to claim 1, wherein said LTA4H inhibitor is a compound of Formula (I), or a pharmaceutically acceptable salt thereof: 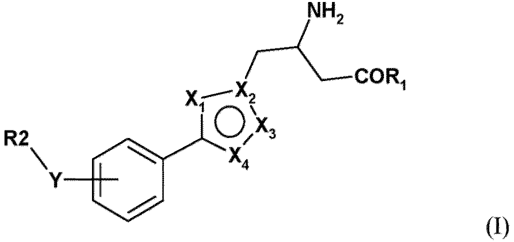 wherein,
R1 is OH or NH₂;
Y is O, S or CH₂;
X1, X2, X3 and X4 are N; or
X1, X2, X3 and X4 are selected from N, NH, C, CH and O with the proviso that at least two of
X1, X2, X3 or X4 are N or NH;
R2 is C₁-C₆ alkyl optionally substituted by phenyl; C₃-C₆ cycloalkyl; phenyl optionally being substituted by halogen, cyano, C₁-C₆ alkyl optionally substituted by halogen, C₁-C₆ alkoxy, or a 5-6 membered heteroaryl ring containing 1 to 3 heteroatoms selected from N, O and S; or a 5 - 10 membered mono- or bicyclic heteroaryl containing 1 to 4 heteroatoms selected from N, O and S, said heteroaryl being optionally substituted by halogen, cyano or C₁-C₆ alkyl optionally substituted by halogen.

3. A LTA4H inhibitor, for use according to claim 1 or 2, wherein said LTA4H inhibitor is (*S*)-3-amino-4-(5-(4-((5-chloro-3-fluoropyridin-2-yl)oxy)phenyl)-2*H*-tetrazol-2-yl)butanoic acid, or a pharmaceutically acceptable salt thereof.

4. A LTA4H inhibitor for use in accordance to any one of claims 1 to 3, wherein the LTA4H inhibitor is disposed in a pharmaceutical formulation, wherein said pharmaceutical formulation comprises one or more pharmaceutically acceptable carriers, each of which is independently selected from a filler, a lubricant, a binder, a desintegrant and a glidant.

5. A LTA4H inhibitor for use in accordance to claim 4, wherein the pharmaceutical formulation is in tablet or capsule form.

6. A pharmaceutical composition comprising a LTA4H inhibitor which is a compound of Formula I, or a pharmaceutically acceptable salt thereof, together with one or more pharmaceutically acceptable carriers for use in the treatment and/or prevention of HS in a patient in need of such treatment and/or prevention.

7. A LTA4H inhibitor for use according to any one of claims 1 to 5 or a pharmaceutical composition for use in accordance to claim 6, wherein the LTA4H inhibitor is administered at a daily dose of about 10 mg to about 100mg.

8. A LTA4H inhibitor for use according to any one of claims 1 to 5 and 7, or a pharmaceutical composition for use in accordance to claim 6 or 7, wherein the LTA4H inhibitor or a pharmaceutical composition comprising it, is administered in combination with one or more second therapeutic agents.

9. A combination product comprising a therapeutically effective amount of a compound of Formula (I) or a pharmaceutically acceptable salt thereof and one or more therapeutic agents, for use in the treatment and/or prevention of HS in a patient in need of such treatment and/or prevention.

10. A LTA4H inhibitor for use according to any one of claims 1 to 5 and 7, a pharmaceutical composition for use according to any one of claims 6 and 7, wherein the patient is additionally treated with at least one topical medication and at least one antiseptic in combination with the LTA4H inhibitor.

11. A LTA4H inhibitor for use according to any one of claims 1 to 5, 7, 8 and 10, a pharmaceutical composition for use according to any one of claims 6 and 7 and 10, or a combination according to claim 9 or 10, wherein, prior to treatment with the LTA4H inhibitor, the patient has not been previously treated with a systemic agent or a topical treatment for HS.

12. A LTA4H inhibitor for use according to any one of claims 1 to 5, 7, 8 and 10, a pharmaceutical composition for use according to any one of claims 6 and 7 and 10, or a combination according to claim 9 or 10, wherein the patient is selected according to one of the following criteria:
a) the patient has moderate to severe HS;
b) prior to treatment with the LTA4H inhibitor, the patient has an HS-PGA score of ≥3;
c) prior to treatment with the LTA4H inhibitor, the patient has at least 3 inflammatory lesions; or
d) prior to treatment with the LTA4H inhibitor, the patient does not have extensive scarring (<10 fistulas) as a result of HS.

13. A LTA4H inhibitor for use according to any one of claims 1 to 5, 7, 8 and 10 to 12, a pharmaceutical composition for use according to any one of claims 6 and 7, and 10 to 12, or a combination according to any one of claims 9 to 12, wherein said patient achieves at least one of the following, e.g. by week 16 of treatment:
a) a simplified HiSCR;
b) a reduction in HS flares;
c) a NRS30;
d) a reduction of ≤ 6 as measured by the DLQI; and/or
e) an improvement in DLQI.

14. A LTA4H inhibitor for use according to any one of claims 1 to 5, 7, 8 and 10 to 12, a pharmaceutical composition for use according to any one of claims 6 and 7, and 10 to 12, or a combination according to any one of claims 9 to 12, wherein at least 40% of said patients achieve a simplified HiSCR; or at least 25% of said patients achieve an NRS30 response; or less than 15% of said patients experience an HS flare, e.g by week 16 of treatment.

15. A LTA4H inhibitor for use according to any one of claims 1 to 5, 7, 8, and 10-12, a pharmaceutical composition for use according to any one of claims 6 and 7, and 10 to 12, or a combination according to any one of claims 9 to 12, wherein the patient has at least one of the following as early as one week after the first dose of the LTA4H inhibitor:
a) a rapid reduction in pain, as measured by VAS or NRS, and
b) a rapid reduction in CRP, as measured using a standard CRP assay.

16. A LTA4H inhibitor for use according to any one of claims 1 to 5, 7, 8 and 10 to 14, a pharmaceutical composition for use according to any one of claims 6 to 8 and 10 to 14, or a combination according to any one of claims 9 to 14, wherein said patient achieves a sustained response after the end of the treatment (for example 3 months after the end of the treatment), as measured by inflammatory lesion count, Hidradenitis Suppurativa Clinical Response (HiSCR), Numerical Rating Scale (NRS), modified Sartorius HS score, Hidradenitis Suppurativa - Physician Global Assessment (HS-PGA), or Dermatology Life Quality Index (DLQI).

17. A LTA4H inhibitor, a pharmaceutical composition, or a combination according to claim 16, wherein said patient achieves a sustained response after the end of treatment, for example 3 months after the end of the treatment, as measured by the simplified HiSCR (sHiSCR).

18. A LTA4H inhibitor for use according to any one of claims 1 to 5, 7, 8 and 10 to 14, a pharmaceutical composition for use according to any one of claims 6 to 8, and 10 to 14, or a combination according to any one of claims 9 to 14, wherein the LTA4H inhibitor is administered at a dose of about 10 mg to about 30mg twice a day.
